# EUROPEAN PATENT SPECIFICATION

(11) **EP 1 773 984 B1**
(45) Date of publication and mention of the grant of the patent: **27.11.2013**
(21) Application number: 05762749.9
(22) Date of filing: 21.07.2005
(51) Int. Cl.: A61L 27/22, A61L 27/56, A61L 27/38, A61K 35/407, A61K 35/32, C12N 5/00

(54) **POROUS PLASMA PROTEIN MATRICES AND METHODS FOR PREPARATION THEREOF**
PORÖSE PLASMAPROTEINMATRIZEN SOWIE VERFAHREN ZUR HERSTELLUNG DAVON
MATRICES POREUSES DE PROTEINES PLASMATIQUES ET LEURS PROCEDES DE PREPARATION

(30) Priority: 22.07.2004 IL 16317904; 22.07.2004 US 895961
(43) Date of publication of application: 18.04.2007
(73) Proprietor: ProChon Biotech Ltd., Nes-Ziona 70400 (IL)
(72) Inventor: YAYON, Avner, 73272 Sitria (IL); BARKAY-OLAMI, Hilla, 75520 Rishon Lezion (IL); AZACHI, Malkit, 76247 Rehovot (IL)
(74) Representative: Becker Kurig Straus
(86) International application number: PCT/IL2005/000782
(87) International publication number: WO 2006/008748

(56) References cited:
- WO-A2-99/15209
- WO-A2-03/007873
- WO-A2-03/007873
- DE-A1- 3 517 456
- US-B1- 6 534 084
- US-B1- 6 534 084

## Description

### FIELD OF THE INVENTION

The present invention relates in general to porous freeze-dried plasma protein biomatrices having open channels useful for clinical applications including as implants for tissue regeneration and tissue engineering. The interconnecting channels, open to the surface, enable cell distribution throughout the biomatrix. The biological and physical characteristics of the matrix are generated by the diffusion of thrombin during formation of the matrix and may be controlled by adjusting the composition and physical properties of the thrombin.

### BACKGROUND OF THE INVENTION

### Tissue Engineering

Tissue engineering may be defined as the art of reconstructing or regenerating mammalian tissues, both structurally and functionally (Hunziker, Osteoarth. Cart. 10:432-63, 2002). Tissue engineering generally includes the delivery of a synthetic or natural scaffold that serves as an architectural support onto which cells may attach, proliferate, and synthesize new tissue to repair a wound or defect.

An example of a tissue that is prone to damage by disease and trauma is the articular cartilage, one of several types of cartilage in the body, found at the articular surfaces of bones. Damage to cartilage may result from an inflammatory disease such as rheumatoid arthritis, from a degenerative process such as osteoarthritis or from trauma such as intraarticular fracture or following ligament injuries. Cartilage lesions are often associated with pain and reduced function and generally do not heal. Without medical intervention, a patient may require total joint replacement.

Current therapeutic strategies for repairing damaged cartilage encompass procedures that induce a spontaneous repair response and those which reconstruct the tissue in a structural and functional manner. The former includes surgical techniques that expose the subchondral bone thereby allowing the infiltration of bone marrow progenitor cells to initiate the healing response. Often the induced tissue is of a mixed fibrocartilage type, is not durable and the clinical improvements are short lived. The latter strategy includes transplantation of chondral or osteochondral cells or tissue from an autologous or an allogeneic source. Autologous Chondrocyte Transplantation (ACT) relies on transplanting into a cartilage lesion autologous chondrocytes, which have been isolated from a patient's cartilage biopsy and expanded *in vitro.* In fact, this technique requires a complicated procedure involving two surgical sites and shows limited clinical success.

Matrices useful for tissue regeneration and/or as biocompatible implants useful for tissue culture are well known in the art. These matrices may therefore be considered as substrates for cell growth either *in vitro* or *in vivo.* Suitable matrices for tissue growth and/or regeneration include both biodegradable and biostable entities. Among the many candidates that may serve as useful matrices claimed to support tissue growth or regeneration are gels, foams, sheets, and porous structures of different forms and shapes.

Typical bioabsorbable materials for use in the fabrication of porous wound dressings or implants include both synthetic polymers and biopolymers such as structural proteins and polysaccharides. The biopolymers may be selected or manipulated to provide greater or lesser degrees of flexibility or susceptibility to degradation.

US Patent No. 5,607,474 discloses a molded biodegradable two-layer implant for repair of defects having two dissimilar tissue types. Each layer is prepared separately and subsequently joined together.

US Patent Nos. 6,306,424; 6,333,029 and 6,534,084 disclose a porous biocompatible foam prepared using a modified polymer-solvent phase separation technique that results in foam having a gradient in stiffness, flexibility, bioabsorption and or pore architecture, associated with a transition in composition. The disclosure teaches foams prepared from synthetic polymers such as aliphatic polyesters.

DE 35 17 456 discloses bone supplement materials consisting of a compressed plasma protein matrix having calcium phosphate particles dispersed therein, and methods of producing the same. For the production of the matrix, plasma protein, calcium phosphate particles and optionally additives are homogenously dispersed in an aqueous solvent. The suspension is then contacted with a cross-linking agent and the resulting gelatine-like mass is compressed utilizing mechanical pressure. Subsequently, the compressed material is then frozen and freeze-dried (cf. abstract). Specifically, this reference describes a homogenous, solid and mechanically stressable moulded padding. No pores and particularly neither a plasma protein concentration gradient nor a size gradient of pores is disclosed.

WO 99/15209 refers to the field of hemostasis, tissue adhesion and support of wound healing and solves the problem of providing means for these applications are based on fibrin and which has a broad filed of application. Disclosed is a fibrin sponge having a specific residual moisture content of between 3 and 35%. The fibrin sponge exhibits a fine porous structure substantially not interspersed by air bubbles. Neither a plasma protein concentration gradient nor a size gradient of pores is disclosed.

### Fibrin

Fibrinogen is a major plasma protein, which participates in the blood coagulation process. Upon blood vessel injury, fibrinogen is converted to insoluble fibrin which serves as the scaffold for a blood clot. Fibrin is known in the art as a tissue adhesive medical device useful for wound healing and tissue repair. Lyophilized plasma-derived protein concentrate (comprising fibrinogen, Factor XIII and fibronectin), in the presence of calcium ions and the serine protease thrombin, forms an injectable biological sealant (fibrin glue). US 5,411,885 discloses a method of embedding and culturing tissue employing fibrin glue.

The fibrin fiber size, density and rate of degradation of thrombin-induced fibrin gels are affected by several different factors. (Carr ME, Thromb. Haemost., 59(3)535-9, 1988; Carr ME and Alving BM, Blood Coag. Fibrinol. 6:567-73, 1995) The factors include fibrinogen source i.e. pure fibrinogen or plasma, fibrinogen, thrombin and factor XIII concentration, ion content, presence of fibronectin, calcium ions and dextran and other factors. In general, fibrin gels having thicker fibrin fibers, which result from low thrombin concentrations, low ionic strength, higher calcium or fibrinogen concentrations undergo fibrinolysis at a faster rate than fibrin gels having thinner fibers. A plasma protein gradient in a fibrin gel or in a porous, plasma protein matrix has not been taught.

US 4,642,120 teaches the use of fibrinogen-containing glue in combination with autologous mesenchymal or chondrocytic cells to promote repair of cartilage and bone defects. US 5,260,420 discloses a method for preparation and use of biological glue comprising plasma proteins for therapeutic use. US 6,440,427 provides an adhesive composition consisting substantially of fibrin forming components and a viscosity-enhancing polysaccharide such as hyaluronic acid.

US 5,972,385 discloses a lyophilized crosslinked collagen-polysaccharide matrix, with optional fibrin, that is administered per se or in combination with therapeutics for tissue repair. US 5,206,023 and 5,368,858 disclose a method and composition for inducing cartilage repair comprising dressing the site with a biodegradable matrix formed by mixing matrix forming material with a proliferative agent and a transforming factor.

A fibrinogen-containing freeze-dried fleece-like structure for use as a wound dressing, filling for bone cavities or support material for release of active materials has been disclosed in US 4,442,655. The structure is prepared by premixing fibrinogen and thrombin solutions, pouring into a mold, freezing and lyophilizing.

A freeze-dried fibrin web for wound healing has been disclosed in US 6,310,267 and 6,486,377. The preparation of said web necessitates a single- or multi-stage dialysis of the fibrinogen solution. According to that disclosure, the single-stage or multistage dialysis of the fibrinogen solution changes crucially its composition by reducing the concentration of salts and amino acids. The dialysis is carried out in an aqueous solution of a physiologically compatible inorganic salt and an organic complexing agent.

US 6,599,515 discloses a porous structure of fibrin or fibrinogen wherein the structure in its substantially dry form, has a compression strain of less than 8%, and a creep modulus higher than 1.5×10⁶ Pa. The mechanical properties are obtained by polymerization of the fibrin or fibrinogen materials in the presence of an amount of a calcium-inhibiting agent, preferably an anticoagulant. After hydration, the structure has a porosity wherein at least 50% by volume of the total porosity is formed by channels with an open cross section of more than 500 µm².

A storage stable fibrin sponge containing a blood clotting activator for hemostasis, tissue adhesion, wound healing and cell culture support is disclosed in US 6,548,729. According to that disclosure, the restoration of moisture or water content following lyophilization is crucial for obtaining a soft, adaptable, absorbent sponge. The sponge may further be impregnated with additives such as a blood clotting activator, stabilizers, preservatives and other agents.

A freeze-dried fibrin clot for the slow release of an antibiotic is described by Itokazu (Itokazu et al., Infection 25:359-63, 1997).

US 5,466,462 and 5,700,476 teach a bioresorbable heteromorphic sponge comprising a biopolymer matrix structure, at least one substructure and at least one pharmacologically active agent. The substructures allow the incorporation of one or more active agents into the final product for phasic release. US 5,443,950 relates to the growth of cells derived from a desired tissue on a pre-established stromal support matrix. US 5,842,477 discloses a method of *in vivo* cartilage repair by implanting a biocompatible, three-dimensional scaffold in combination with periosteal/perichondrial tissue and stromal cells, with or without bioactive agents. US 6,569,172 discloses an implantable article for cartilage repair comprising a support matrix, and a mixture of chondrocyte cells and adhesive adhered to an edge of said support matrix.

PCT patent application WO 03/079985 teaches a method of preparing a biomimetic scaffold comprising the steps of providing two or more bio-ink solutions and codepositing said bio-ink solutions to create the scaffold using solid free-form fabrication (SFF). SFF are computer-aided design and manufacturing methods that can fabricate automatically complex shapes directly from computerized models. SFF processes rely on a layered manufacturing paradigm that builds shapes by incremental material deposition and fusion of thin cross-sectional layers. Fibrinogen, thrombin and collagen are disclosed as examples of structural bio-inks. A scaffold having a patterned three-dimensional spatial and/or concentration gradient of therapeutic or structural elements is cited, yet a uniform matrix having a self-assembled continuous plasma protein gradient is not taught.

PCT patent application WO 03/007873 by some of the applicants of the present invention discloses a fibrin matrix comprising plasma proteins and at least one anti-fibrinolytic agent, optionally further comprising agents such as polysaccharides, anionic polysaccharides, glycosaminoglycans, or synthetic polymers added in the preparation to improve certain physical, mechanical and biological properties of the matrix. Copending international patent application PCT/IL2004/000088 by some of the applicants of the present invention teaches a porous plasminogen-free plasma protein sponge and a method of preparing the sponge. The sponge may be prepared by sequential transferring of the thrombin solution and plasma protein solution into a mold or solid receptacle followed by freezing the clotted mixture and lyophilizing or alternatively, premixing the plasma protein solution with thrombin solution and casting into a mold or support prior to achieving clotting; the clotted mixture is frozen and lyophilized.

### Collagen

Collagen is the most abundant protein in the body and constitutes a major part of the extracellular matrix. Collagen matrices and sponges useful for tissue regeneration are well known in the art. PCT publication WO 96/24310 discloses a multistage collagen based template or implant characterized by a first layer comprising a dense collagen membrane secured to a second layer comprising a porous collagen matrix.

US 4,837,379 discloses a fibrin-collagen tissue equivalent comprising (i) a hydrated collagen lattice contracted by a contractile agent, such as fibroblasts, and (ii) fibrin. According to that patent, the tissue equivalents are prepared either by casting the collagen and fibrin lattice together or by incorporating the fibrin into the collagen lattice after the lattice is formed. Alternatively, a layered tissue equivalent may be formed.

A porous collagen structure impregnated with a slow setting fibrin adhesive at a fibrin adhesive to collagen volume proportion of at least 1 to 4, useful for osteocartilaginous reconstruction, has been disclosed in WO 93/16739. A method of producing a lyophilized tissue adhesive useful for wound healing based on collagen and fibrin is taught in US 4,600,574. The method comprises the steps of (a) impregnating a tissue compatible flat material selected from collagen, gelatin and polysaccharide with a solution comprised of fibrinogen and factor XIII, and (b) lyophilizing said impregnated flat material to obtain a coherent matrix of said tissue-compatible flat material. Preferably, a porous non-woven fabric is used.

There remains an unmet need for a natural, three-dimensional matrix for use in tissue regeneration and repair that integrates a matrix having favorable pore size, pore distribution and interconnected channels for cell maintenance and nutrient diffusion while it provides a structural support.

### SUMMARY OF THE INVENTION

The present invention provides a lyophilized biomatrix comprising plasma proteins and thrombin, wherein the crosslinked plasma proteins obtained by the action of thrombin are present in a continuous concentration gradient along at least one axis of the matrix and form porous structures. The concentration gradient thus obtained is a result of the diffusion of thrombin through the plasma protein solution. It is now disclosed that the open channel structures, interchangeably referred to as biomatrices, matrices, sponges and scaffolds, have unexpected advantageous biological and physical properties. These biomatrices are particularly beneficial for supporting well-distributed cell growth and are useful for a variety of biotechnological and medical applications.

By generating a diffusion gradient of a natural enzyme, e.g. thrombin, the inventors of the present invention have produced, for the first time, biocompatible matrices comprising natural plasma proteins having a continuous gradient in composition and excellent scaffold architecture without resorting to the use of synthetic polymers and complex production methods to achieve these features.

According to a first aspect, the present invention provides a porous, freeze-dried plasma protein matrix comprising plasma proteins and thrombin, having two opposing surfaces substantially parallel to the horizontal axis of the matrix and at least one additional surface extending along the periphery of the sponge substantially parallel to the vertical axis, wherein the plasma proteins crosslinked by the action of thrombin are present in a gradient having a higher concentration along one of the opposing surfaces, and wherein the average size of the pores is smaller along the surface of the matrix exposed to the higher concentration of thrombin.

According to one embodiment the thrombin is provided at a concentration of 300 IU/ml to 1,500 IU/ml. According to another embodiment the thrombin is provided at a concentration of 500 IU/ml to 1000 IU/ml. According to a further embodiment the plasma proteins and thrombin are present in a ratio of 5:1 (v/v) to 50:1 (v/v). According to yet another embodiment the plasma proteins and thrombin are present in a ratio of 8:1 (v/v) to 30:1 (v/v). According to yet a further embodiment the pores having an average size of 5 µm to 30 µm in cross section in the fraction of the matrix exposed to the higher concentration of thrombin. According to another embodiment the pores having an average size of 10 µm to 20 µm in cross section in the fraction of the matrix exposed to the higher concentration of thrombin. According to a further embodiment the plasma proteins are substantially devoid of plasminogen. According to yet another embodiment the plasma proteins are substantially devoid of organic chelating agents. According to yet a further embodiment at least one of the plasma proteins is autologous. According to another embodiment at least one of the plasma proteins is recombinant. According to a further embodiment the protein matrix further comprises a fibrin fiber-modifying agent. According to yet a further embodiment the protein matrix has any suitable geometric shape. According to yet another embodiment the plasma proteins crosslinked by the action of thrombin are generated by a thrombin solution comprising a transglutaminase; preferably wherein the transglutaminase is factor XIII.

According to another embodiment the plasma proteins crosslinked by the action of thrombin are generated by a thrombin solution comprising at least one viscosity-enhancing agent selected from a protein, a glycolsaminoglycan, a polysaccharide, disaccharide and a synthetic polymer. Preferably the protein is an extracellular matrix protein selected from collagen, fibronectin, elastin and laminin. More preferably the protein is collagen or the polypeptide is fibronectin. Also preferably the glycosaminoglycan is selected from crosslinked hyaluronic acid and non- crosslinked hyaluronic acid.

According to another embodiment the porous freeze-dried plasma protein matrix further comprises at least one additive selected from the group consisting of a polysaccharide, a glycosaminoglycan and a synthetic polymer. Preferably the glycosaminoglycan is selected from crosslinked hyaluronic acid, non-crosslinked hyaluronic acid and heparin and derivatives thereof.

According to a further embodiment the porous freeze-dried plasma protein matrix further comprises at least one bioactive agent selected from the group consisting of growth factors, cytokines, platelets, platelet supernatant and platelet derived proteins, hormones, analgesics, anti-inflammatory agents, anti-microbials and enzymes. Preferably the growth factor is selected from a fibroblast growth factor and variant thereof.

According to yet another embodiment the porous freeze-dried plasma protein matrix further comprisies cells. Preferably the cells are selected from stem cells, progenitor cells, chondrocytes, osteoblasts, hepatocytes, mesenchymal cell types, endothelial cell types, epithelial cell types, urothelial cell types, endocrinal cell types, neuronal cell types, pancreatic cell types, renal cell types and ocular cell types. More preferably the cells are chondrocytes or hepatocytes.

According to a second aspect the present invention provides a method of preparing a porous, freeze-dried plasma protein matrix comprising plasma proteins and thrombin, having two opposing surfaces substantially parallel to the horizontal axis of the matrix and at least one additional surface extending along the periphery of the sponge substantially parallel to the vertical axis, wherein the plasma proteins crosslinked by the action of thrombin are present in a gradient having a higher concentration along one of the opposing surfaces and, wherein the average size of the pores in cross section is smaller along the surface of the matrix exposed to the higher concentration of thrombin comprising the steps: introducing a thrombin solution to a solid receptacle or mold; layering a plasma protein solution over the thrombin solution in the solid receptacle or mold; incubating under conditions appropriate to achieve clotting; freezing the clotted mixture; and lyophilizing the clotted mixture, to obtain a porous matrix.

According to one embodiment the further comprises the steps of: seeding the porous matrix with cells; and implanting said cell-bearing porous matrix into an individual in need thereof. According to another embodiment the thrombin solution comprises thrombin at a concentration of 300 IU/ml to 1,500 IU/ml. According to a further embodiment the thrombin the thrombin solution comprises thrombin at a concentration 500 IU/ml to 1000 IU/ml. According to yet another embodiment the plasma protein solution and thrombin solution are provided in a ratio of 5:1 (v/v) to 50:1 (v/v). According to yet a further embodiment the plasma protein solution is substantially devoid of plasminogen. According to another embodiment the plasma protein solution is substantially devoid of organic chelating agents. According to a further embodiment at least one of the plasma proteins is autologous. According to yet another embodiment at least one of the plasma proteins is recombinant. According to yet a further embodiment the porous freeze-dried plasma protein matrix further comprises an agent affecting fibrin fiber thickness, wherein the agent is selected from the group consisting of: calcium ions, a salt that alters ionic strength, a serine protease activator, a serine protease inhibitor and dextran sulphate. According to yet another embodiment the receptacle or mold having any suitable shape.

According to a further embodiment the plasma protein solution and thrombin solution are provided in a ratio of 8:1 (v/v) to 30: 1 (v/v). Preferably the thrombin solution further comprising at least one viscosity-enhancing agent selected from a protein, a glycosaminoglycan, a polysaccharide, disaccharide and a synthetic polymer. More preferably the protein is an extracellular matrix protein selected from collagen fibronectin, elastin and laminin. Most preferably the protein is collagen or the polypeptide is fibronectin. Also preferably the glycosaminoglycan is selected from crosslinked hyaluronic acid and non-crosslinked hyaluronic acid.

According to another embodiment the thrombin solution comprising a transglutaminase, wherein the transglutaminase is preferably factor XIII.

According to a further embodiment the plasma protein solution further comprises at least one additive selected from the group consisting of a polysaccharide, a glycosaminoglycan and a synthetic polymer. Preferably the at least one additive is a glycosaminoglycan. More preferably the glycosaminoglycan is selected from crosslinked and non-crosslinked hyaluronic acid, or the glycosaminoglycan is selected from heparin and a derivative thereof.

According to yet another embodiment the plasma protein solution further comprises at least one bioactive agent selected from the group consisting of growth factors, cytokines, platelets, platelet supernatant and platelet derived proteins, hormones, analgesics, anti-inflammatory agents, anti-microbials and enzymes. Preferably the growth factor is selected from a fibroblast growth factor and variant thereof.

According to another embodiment when the method further comprises the steps of: seeding the porous matrix with cells; and implanting said cell-bearing porous matrix into an individual in need thereof, the cells are selected from stem cells, progenitor cells, chondrocytes, osteoblasts, hepatocytes, mesenchymal cell types, endothelial cell types, epithelial cell types, urothelial cell types, endocrinal cell types, neuronal cell types, pancreatic cell types, renal cell types and ocular cell types. Preferably the cells are chondrocytes or hepatocytes.

According to a third aspect the present invention provides a porous, freeze-dried plasma protein matrix as detailed above for use in the treatment of diseased or injured tissue. Preferably the diseased or injured tissue is selected from neural, muscular, skeletal, cartilaginous, tendonous, hepatic, pancreatic, renal, ocular, arteriovenous, mammary and urinary tissue types; preferably cartilage tissue or hepatic tissue.

The plasma proteins are purified or partially purified and are obtained from total blood, blood fractions, blood derivative, cryoprecipitate, recombinant proteins, plasma and plasma fractions. According to one example the plasma proteins are obtained from a commercially available source, including native or recombinant proteins. The plasma proteins may be selected from xenogeneic, allogeneic and autologous plasma sources. In some examples the plasma source is autologous plasma. The plasma proteins may substantially be devoid of organic chelating agents. The plasma proteins may substantially be devoid of plasminogen.

The plasma proteins may further comprise at least one additive that imparts additional advantageous biological, physical and mechanical characteristics to the matrix. The at least one additive may be selected from the group consisting of calcium salts, glycosaminoglycans, polysaccharides, and synthetic polymers.

It is now disclosed that the properties of the matrix, including pore size or diameter and biodegradability may be controlled by varying the properties of the thrombin solution. The properties of the thrombin solution that may be varied include temperature, viscosity, volume, composition and concentration.

The porous freeze-dried plasma protein matrix of the present invention is useful in treating orthopedic defects *inter alia* articular cartilage lesions arising from trauma such as an accident or sports injury or disease such as osteoarthritis. In one example the porous plasma protein matrix comprises autologous plasma proteins and autologous chondrocytes.

The porous freeze-dried plasma protein matrix of the invention is useful, *inter alia,* as an unexpectedly advantageous support for cellular growth. In one example the matrix, either *per se* or cell-bearing, is useful in reconstructive surgery, for example as a matrix for regenerating and or repairing tissue. In one example, the matrix is inoculated or seeded with cells and the cells are allowed to proliferate *in vitro* prior to *in vivo* implantation. In another example, the matrix is seeded with cells that have been cultured or harvested and the sponge comprising the cells is implanted *in situ.* In one example the matrix is implanted to the defect site prior to cell seeding. In one example the matrix is useful for delivering cells for gene therapy.

These and further embodiments will be apparent from the Figures, detailed description, examples and claims that follow.

### BRIEF DESCRIPTION OF THE FIGURES

The present invention will be understood and appreciated more fully from the following detailed description taken in conjunction with the figures in which:
Figures 1A and 1B are photographs of a 35 mm diameter matrix. Figure 1A shows the dry matrix while Figure 1B shows a cell-bearing matrix in medium. Figure 1C shows a cell-bearing sponge following implantation into an irregular shaped defect that was introduced into the articular cartilage of a pig's knee (arrow).
Figure 2A shows a histological section a plasma protein matrix comprising a collagen matrix on one opposing surface. The arrow indicates the collagen matrix. Figure 2B shows a histological section matrix comprising calcium-phosphate particles. The arrows denote some of the particles.
Figures 3A and 3B show scanning electron microscope (SEM) photographs of a mixed plasma protein matrix prepared by premixing the thrombin and plasma proteins.
Figures 4A-4B show SEM photographs of the nonmixed matrix. Figure 4A shows the matrix periphery and Figure 4B shows the top center of the matrix.
Figure 5A-5D show SEM photographs of the matrix of the invention. Figures 5A, 5B and 5C show the surface of the matrix exposed to a lower thrombin concentration; Figure 5D shows the surface of the matrix exposed to a higher thrombin concentration. The arrows indicate pores.
Figure 6 shows a graph of cell survival in several matrices of the invention following three day incubation.
Figure 7A and 7B show cross sections of a matrix of the invention, seeded with chondrocytes.
Figures 8A and 8B show cross sections of a cell-bearing matrix of the invention, seeded with rat hepatocytes. Figure 8A shows the cells within the matrix after an three day incubation, Figure 8B shows the cells in the matrix following a two week incubation.

### DETAILED DESCRIPTION OF THE INVENTION

Though numerous biomatrices comprising plasma or tissue proteins are known in the art to which the present invention pertains, none has proven entirely satisfactory in meeting the criteria required for successful tissue engineering and tissue repair. The present invention provides a lyophilized biomatrix comprising plasma proteins and thrombin, wherein the plasma proteins crosslinked by the action of thrombin are present in a concentration gradient along at least one axis of the matrix, provides a structure having unexpected advantageous physical, mechanical and biological properties. Without wishing to be bound to theory the features of the matrix are generated by a gradient of thrombin through the plasma proteins. Thrombin is acting in a concentration gradient from one surface to the opposing surface. Accordingly, the matrix obtained may have a stepwise gradient or a continuous gradient of plasma proteins that are crosslinked by the action of thrombin. Alternatively, the matrix may comprise separate layers wherein one layer comprises a higher concentration of plasma proteins that are crosslinked by the action of thrombin than another second layer.

The advantageous physical and mechanical properties include:
dense structural support provided by the action of a high concentration of thrombin along one surface;
excellent microarchitecture including continuous open pore channels for optimal cell seeding, three dimensional cell distribution and rapid equilibrium of solutes, bioactive materials and waste products;
pliability for safe and easy handling and uncomplicated implantation.

The advantageous biological properties of the matrix include
biocompatible, non-immunogenic and biodegradable natural products;
excellent cell attachment and cell distribution throughout the matrix;
excellent cell proliferation and or differentiation, useful for tissue regeneration and repair;
may be formulated for controlled release of bioactive agents;
plasma proteins may be retrieved from autologous or recombinant material thereby obviating the need for pooled blood sources with the attendant health risks.

In addition, the matrix is prepared using easily accessible materials and according to a simple protocol.

The compositions and methods of the present invention are effective for *in vitro* and *in vivo* applications including as cell-bearing implants for tissue engineering and repair.

The matrices of the invention provide all components fundamental for tissue repair, thus facilitating the medical practitioner's task. In addition, the composition of the sponge renders it suitable for minimally invasive surgery of articular cartilage. The sponge may be implanted in a mini-arthrotomy or arthroscopy procedure, thus obviating the need for multiple site surgeries and a full arthrotomy, the standard procedures for ACT.

### Definitions

For convenience and clarity certain terms employed in the specification, examples and claims are described herein.

A "biomatrix" as used herein, refers to a porous structure, solid or semi-solid biodegradable substance having pores and interconnecting channels sufficiently large to allow cells to populate, or invade the matrix. The term biomatrix may be used interchangeably with matrix, sponge or scaffold. The plasma protein matrix of the invention wherein the plasma proteins that are crosslinked by the action of thrombin are present in a gradient and that the pores that are present in the fraction of the matrix exposed to a higher thrombin concentration have a smaller diameter and are less abundant than those exposed to a lower thrombin concentration. The matrix-forming components of plasma include fibrinogen and crosslinking agent including Factor XIII, require addition of a cleaving agent, such as thrombin in the presence of bivalent calcium ions, to form a clot. The clot is subsequently freeze-dried yielding a porous plasma protein matrix having interconnecting channels which open to the surface of the matrix.

The plasma protein matrix of the present invention may comprise fibrinogen and or fibrin monomers and or crosslinked fibrin. Fibrinogen is broken down into fibrin monomers by thrombin. Factor XIII, which becomes activated by thrombin in the presence of calcium ions, subsequently forms covalent links between the carboxyl and amino groups of the fibrin monomers to form crosslinked fibrin.

The plasma protein matrix of the present invention is useful as an implant *per se,* for the culturing of cells or as a cell-bearing tissue replacement implant. Although the examples presented herein refer to the use of the matrix in cartilage and liver repair, it is to be understood that the matrix may be used for tissue reparation and regeneration of many other tissue types including bone, mammary, epithelial, neural, pancreatic and endothelial tissue types.

"Plasma" as used herein refers to the fluid, non-cellular portion of the blood of humans or animals as found prior to coagulation.

"Plasma protein" as used herein refers to the soluble proteins found in the plasma of humans or animals. These include but are not limited to coagulation proteins, albumin, lipoproteins and complement proteins. The major plasma protein is fibrinogen, which upon cleavage, physiologically by thrombin but pathologically by other substances, is converted to fibrin monomers. The fibrin monomers are crosslinked by a transglutaminase, including FactorXIII, to form a stable clot. The term "fibrin matrix" may be used interchangeably with a "plasma protein matrix".

As used herein the term "plasminogen" refers to plasminogen and plasmin. The terms "Substantially devoid of plasminogen" or "plasminogen-free" refer to plasma proteins having less than about 20% plasminogen normally present in plasma, preferably less than about 10% plasminogen normally present in plasma, preferably less than about 5% of the plasminogen normally present in plasma. Plasma normally compromises about 200 mg plasminogen per liter fresh plasma (about 2 µmol/liter).

Factor XIII, is an enzyme of the coagulation cascade which serves to stabilize fibrin by crosslinking the adjacent gamma-chain C-termini of fibrin clots. Although Factor XIII is the preferred crosslinking agent of the present invention, according to certain embodiments the crosslinking agent may be selected from other agents including different types of transglutaminases.

A "substantial absence of organic chelating agents" or "substantially devoid of organic chelating agents" refers to a concentration of less than 1 mm of an organic chelating agent such as EDTA or other organic chelating agents known in the art.

"Platelet rich plasma" or "PRP" as used herein refers to plasma containing platelets. A platelet sample or platelet-derived extract or supernatant may be added exogenously. Alternatively, platelet-rich plasma may serve as the source for plasma proteins. Methods for preparing platelet rich plasma are taught in US 6,475,175 and US 6,398,972.

The term "cell-bearing" as used herein refers to the capacity of the matrix to allow cells to be maintained within its structure. In one embodiment, the cells are able to invade the pores and channels of the matrix and may undergo proliferation and or differentiation.

The term "stem cell" as referred to herein refers to an undifferentiated cell that is capable of proliferation. Stem cells are capable of producing either new stem cells or cells called "progenitor cells" that differentiate to produce the specialized cells found in mammalian tissue and organs.

The term "biocompatible" as used herein refers to materials which have low toxicity, clinically acceptable levels of foreign body reactions in the living body, and affinity with living tissues.

The terms "lyophilize" or "freeze drying" refer to the preparation of a composition in dry form by rapid freezing and dehydration in the frozen state (sometimes referred to as sublimation). This process may take place under vacuum at reduced air pressure resulting in drying at a lower temperature than required at full pressure.

The term ``residual moisture" as used herein refers to the amount of moisture remaining in the dried sample. It is referred to as a percent of the weight of the sample. In one embodiment the matrices of the invention have less than 15% residual moisture, preferably less than 10% and more preferably less than 5% residual moisture. The lyophilized matrix may be stored under conditions that preserve its moisture level.

The term "implantation" refers to the insertion of a sponge of the invention into an individual, whereby the implant serves to replace, fully or partially, tissue that has been damaged, diseased or removed.

The "biologically active" or "bioactive agents" incorporated into the sponge, for example, growth factors, platelet and platelet extracts, angiogenic factors, and the like, are advantageous to, in a non-limiting example, encourage a more rapid growth or differentiation of the cells within the implant, or a more rapid vascularization of the implant. Such factors have now been shown to be effectively retained within the sponge and form a source, or depot, of bioactive agent, for sustained release for *in vivo* or *in vitro* applications. Other bioactive agents include antibiotics, enzymes, additional plasma proteins or mixtures thereof.

It is to be noted that the pores within the biomatrices of the present invention may be round, elliptical or any random shape. The pore size is easily determined from SEM photographs of the surfaces of the matrices. Thus "pore size" or "pore diameter" as referred to herein is determined by measuring the diameter of a pore in cross section in one axis (d1) of a pore and the diameter of the perpendicular axis (d2) of a dried matrix and are presented either as an average of the two measurements or as "d1xd2".

"Polysaccharides" as used herein refer to complex carbohydrates made of more than one saccharide. Included in the definition are anionic polysaccharides, including non-modified as well as chemical derivatives thereof, that contains one negatively charged group (e.g., carboxyl groups at pH values above about 4.0) and includes salts thereof, such as sodium or potassium salts, alkaline earth metal salts such as calcium or magnesium salts. Non-limiting examples of anionic polysaccharides include pectin, alginate, galactans, galactomannans, glucomannans and polyuronic acids.

A "glycosaminoglycan" or "GAG" as used herein refers to a long unbranched polysaccharide molecules found on the cell surface or extracellular matrix. Non-limiting examples of glycosaminoglycan include heparin, chondroitin sulfate, dextran sulfate, dermatan sulfate, heparan sulfate, keratan sulfate, crosslinked or non-crosslinked hyaluronic acid, hexuronyl hexosaminoglycan sulfate, and inositol hexasulfate. Derivatives, salts and mimetics of the above, including low molecular weight heparin are intended to be included in the invention. Without wishing to be bound to theory, the presence of certain GAGs, in particular heparin, aids in immobilizing heparin binding growth factors such as those of the Fibroblast Growth Factor (FGF) family.

The term "cartilage" as used herein, refers to a specialized type of connective tissue that contains chondrocytes embedded in an extracellular matrix. The biochemical composition of cartilage differs according to type but in general comprises collagen, predominantly type II collagen along with other minor types, e.g., types IX and XI, proteoglycans, other proteins and water. Several types of cartilage are recognized in the art, including, for example, hyaline cartilage, articular cartilage, costal cartilage, fibrous cartilage (fibrocartilage), meniscal cartilage, elastic cartilage, auricular cartilage, and yellow cartilage. The production of any type of cartilage is intended to fall within the scope of the invention. The term "chondrocytes," as used herein, refers to cells which are capable of producing components of cartilage tissue.

The term "variant" as used herein refers to a polypeptide sequence that possesses some modified structural property of the wild type or parent protein. For example, the variant may be truncated at either the amino or carboxy terminus- or both termini or may have amino acids deleted, inserted or substituted. It may be antagonistic or agonistic with respect to normal properties of the native protein. The variant may have similar or altered activity as compared to that of the wild type protein.

Disclosed is a porous, freeze-dried plasma protein matrix comprising plasma proteins and thrombin, having two opposing surfaces substantially parallel to the horizontal axis of the matrix and at least one additional surface extending along the periphery of the sponge substantially parallel to the vertical axis; wherein the plasma proteins crosslinked by the action of thrombin are present in a gradient having a higher concentration along one of the opposing surfaces and, wherein the average size of the pores in cross section is smaller along the surface of the matrix exposed to the higher concentration of thrombin.

The thrombin may be provided in a in a solution having a concentration of 300 IU/ml to 1,500 IU/ml, preferably at 500 to 1000 IU/ml.

The porous freeze-dried plasma protein matrix comprises plasma proteins and thrombin in a ratio of 5:1 (v/v) to 50:1 (v/v), preferably in a ratio of 8:1 to 30:1, more preferably 12:1 to 15:1. Several factors including application and desired rate of degradation may be considered in choosing the preferred plasma protein to thrombin ratio and the plasma protein and thrombin concentrations.

The thrombin may further comprise a viscosity-enhancing agent that may be selected from at least one glycosaminoglycan, a protein, a polysaccharide, disaccharide and a synthetic polymer. The protein is preferably selected from a soluble protein such as albumin or an extracellular matrix protein including collagen, elastin, laminin and fibronectin. A combination of two or more of the proteins may be incorporated into the matrix. The GAG may be selected from crosslinked hyaluronic acid, non-crosslinked hyaluronic acid, chondroitin sulfate, dextran sulfate, dermatan sulfate, a syndecan and keratan sulfate. In one embodiment the thrombin comprises non-crosslinked hyaluronic acid at a final concentration (v/v) of 0.005% to 0.05%, preferably at a final concentration of 0.01% to 0.03%. Without wishing to be bound by theory, the presence of a viscosity-enhancing agent may affect the diffusion rate of the thrombin through the plasma proteins.

The thrombin may comprise at least one therapeutic protein, including growth factors. The therapeutic protein may be a heparin binding protein selected from the family of fibroblast growth factors (FGF), and their variants.

The plasma proteins may further comprise at least one agent that affects fibrin fiber thickness, i.e. an agent including calcium, a salt that alters ionic strength, a serine protease activator, a serine protease inhibitor and dextran sulfate. Without wishing to be bound by theory the thickness of the fibrin fibers may determine the rate of matrix degradation. In general, a thicker fibrin fiber degrades faster than a thinner fibrin fiber. For example, when fibrin is formed from fibrinogen at an ionic strength greater than that of normal plasma a thinner fibrin fiber is formed. In contrast, fibrin fibers formed in the presence of dextran sulfate are thicker than control. (Carr, ME and Alving, BM, Blood Coag. Fibrin., 6:567-573, 1995). The plasma proteins may comprise CaCl₂ at a final concentration of 5 mM to 50 mM.

The matrix may be in direct contact with or apposed to a membrane such as a natural polypeptide or synthetic membrane. Incorporation of a membranous layer during preparation of the matrix may increase mechanical strength of the matrix and or may allow for the use of sutures, staples or various fixation devices to hold the matrix in place.

The matrix comprises plasma proteins, the plasma proteins being fibrinogen or fibrin or a combination of both and a crosslinking agent, preferably Factor XIII. In one embodiment of the invention, the plasma proteins comprise factor XIII at a final concentration of 1 IU/ml to 10 IU/ml, preferably at a final concentration of 2 IU/ml to 4 IU/ml.

The plasma proteins may be obtained from total blood, blood fractions, blood derivative, cryoprecipitate, recombinant proteins, plasma and plasma fractions. According to one embodiment the plasma proteins are obtained from a commercially available source, including native or recombinant proteins. The plasma proteins may be selected from xenogeneic, allogeneic and autologous plasma sources. In certain applications, including cartilage repair, an autologous plasma source is preferred. Some or all of the plasma proteins may be autologous. The plasma proteins may substantially be devoid of organic chelating agents.

The plasma proteins may substantially be devoid of plasminogen. Freeze-dried plasma protein matrices substantially devoid of plasminogen have been disclosed in copending PCT application PCT/IL2004/000088 of some of the inventors of the present application. Plasminogen may be removed from the plasma by methods known in the art. PCT publication WO 02/095019 discloses a method for specifically removing plasminogen and plasmin in the presence of fibrinogen from a mixture such as blood or cryoprecipitate. PCT publication WO 95/25748 discloses a topical fibrinogen complex essentially free of plasminogen whereby the plasminogen was removed using a Sepharose™ -lysine column. Alternatively, some or all of the plasma proteins may be recombinant and consequentially devoid of plasminogen, for example as disclosed in PCT publication WO 99/56797.

The plasma proteins, specifically fibrinogen, is meant to include fibrinogen variants, including the high molecular weight (HMW), the low molecular weight (LMW) and the LMW derivative (LMW') variants, for example as disclosed in WO 03/087160.

The matrix of the invention may further comprise additives that impart other advantageous biological, physical and Mechanical characteristics to the matrix. Copending PCT patent application WO 03/007873 of some of the inventors of the present invention discloses a fibrin matrix comprising plasma proteins and at least one anti-fibrinolytic agent, optionally further comprising agents such as polysaccharides, anionic polysaccharides, glycosaminoglycans (GAG), or synthetic polymers to improve certain physical, mechanical and biological properties of the matrix.

The GAG may be selected from crosslinked hyaluronic acid, non-crosslinked hyaluronic acid, heparin and heparin derivatives and heparin mimetics, chondroitin sulfate, dextran sulfate, dermatan sulfate, heparan sulfate and keratan sulfate. The non-crosslinked hyaluronic acid may be present in a final concentration of 0.05% to 0.5% (V/V) more preferably 0.075% to 0.125%. The crosslinked hyaluronic acid may be present in a final concentration of 0.001% to 0.1% and more preferably 0.05% to 0.09% (VN).

One example may further include the incorporation of an additional synthetic or natural polymer prior to formation of the clot which may modify certain properties of the sponge including physical, mechanical and/or biological properties. These may impart superior characteristics including elasticity, cell aattachment, open channels and strength to the sponge. Non-limiting examples of natural polymers include cellulose, pectin, polyuronic acids, hexuronyl hexosaminoglycan sulfate and inositol hexasulfate.

The synthetic polymers useful for the present invention may be non-biodegradable or biodegradable. Examples of non-degradable materials include polytetrafluoroethylene, perfluorinated polymers such as fluorinated ethylene propylene, polypropylene, polyethylene, polyethylene terapthalate, silicone, silicone rubber, polysufone, polyurethane, non-degradable polycarboxylate, non-degradable polycarbonate, non-degradable polyester, polyacrylic, polyhydroxymethacrylate, polymethylmethacrylate, polyamide such as polyesteramide, and copolymers, block copolymers and blends of the above materials.

Non-limiting examples of degradable materials include hydrolyzable polyesters such as polylactic acid and polyglycolic acid, polyorthoesters, degradable polycarboxylates, degradable polycarbonates, degradable polycaprolactones, polyanhydride, and copolymers, block copolymers and blends of the above materials. Other components include surfactants including lecithin.

Also disclosed is a heterogeneous sponge comprising particulate matter such as calcium phosphate crystals or other particles. The particulate matter may be incorporated *ab initio* in order to provide a matrix having physical or biological characteristics advantageous for certain applications.

### Bioactive Agents

The matrix of the invention may further comprise at least one bioactive agent, such as a cytokine, a growth factor and their activators, platelets, a bioactive peptide etc. Without wishing to be bound by theory, incorporation of such agents into the sponge of the present invention provides a slow-release or sustained-release mechanism. Sustained release of a bioactive agent may depend on a variety of factors including growth factor concentration, type of glycosaminoglycan incorporated and the concentration of plasma proteins and thrombin.

Without wishing to be bound to theory, as the matrix degrades *in vivo,* the bioactive agents are released into the surrounding milieu. For example, growth factors, structural proteins or cytokines which enhance the temporal sequence of wound repair, enhance angiogenesis, alter the rate of proliferation or increase the metabolic synthesis of extracellular matrix proteins are useful additives to the matrix of the present invention. The bioactive proteins of the invention are polypeptides or derivatives or variants thereof, obtained from natural, synthetic or recombinant sources, which exhibit the ability to stimulate DNA synthesis and or cell division and or differentiation of a variety of cells, including primary fibroblasts, embryonal stem cells (ESC), adult stem cells, chondrocytes, vascular and corneal endothelial cells, osteoblasts, myoblasts, smooth muscle, neuronal cells and other cell types. Representative proteins include bone growth factors (BMPs, IGF) and fibroblast growth factors and their variants, including FGF2, FGF4, FGF9 and FGF 18 for bone and cartilage healing, cartilage growth factor genes (CGF, TGF-β) for cartilage healing, nerve growth factor genes (NGF) and certain FGFs for nerve healing, and general growth factors such as platelet-derived growth factor (PDGF), vascular endothelial growth factor (VEGF), insulin-like growth factor (IGF-I), keratinocyte growth factor (KGF), endothelial derived growth supplement (EDGF), epidermal growth factor (EGF) and other proteins which may enhance the action of the growth factors including heparin sulfate proteoglycans (HSPGs) their mimetics such as dextran sulfate, sucrose octa sulfate or heparin, and fragments thereof. Other factors shown to act on cells forming bone, cartilage or other connective tissue include retinoids, growth hormone (GH), and transferrin. Proteins specific for cartilage repair include cartilage growth factor (CGF), FGFs and TGF-β. Growth factors important for liver regeneration and repair include hepatocyte growth factor, Tufα, interleukin-6, EGF and others. In certain embodiments, the FGF is an FGF having the capacity to induce or enhance liver regeneration, cartilage and bone repair and regeneration and or angiogenesis.

The matrix may further include one or more of the following biologically active agents: blood platelets, platelet supernatants or extracts and platelet derived proteins; antiseptics, such as methylene blue, and/or one or more drugs including antimicrobials such as antibiotics and antiviral agents; chemotherapeutic agents; anti-rejection agents; analgesics and analgesic combinations; anti-inflammatory agents; enzymes; extracellular matrix protein or adhesion proteins and hormones such as steroids.

Bioactive agents including platelets and platelet supernatant or extract promote the proliferation and differentiation of skeletal cells including chondrocytes and osteoblasts and of other cell types including but not limited to hepatocytes and endothelial cells. Bioactive agents belonging to the class of anti-microbial or anti-inflammatory agents may accelerate the healing process by minimizing infection and inflammation. Enzymes such as chondroitinase or matrix metalloproteinases (MMPs) may be incorporated to aid in the degradation of cartilage, thus stimulating release of cells from the tissue into the matrix and the surrounding milieu.

The growth factors and other bioactive agents may be incorporated at a wide range of concentrations, depending on the application. For certain applications sustained release of a bioactive agent is preferred. Sustained release of a bioactive agent may depend on several factors including growth factor concentration, type of glycosaminoglycan incorporated and plasma protein and thrombin concentration.

Also disclosed is a porous freeze-dried plasma protein matrix further comprising at least one glycosaminoglycan and at least one bioactive agent, wherein the at least one glycosaminoglycan is heparin and the at least one bioactive agent is a therapeutic protein belonging to the FGF family of growth factors or a variant thereof. This sponge provides phasic release of the FGF from the matrix and may be beneficial in certain therapeutic applications.

Additionally, cells genetically engineered to express the aforementioned therapeutic proteins or peptides including anti-inflammatory peptides or proteins, growth factors having angiogenic, chemotactic, osteogenic or proliferative effects are included in the present invention. In a non-limiting example, for cartilage repair cells may be transfected with genes selected from a group including transforming growth factor-β (TGF-β), certain FGFs or CGF; for bone repair periosteal or other mesenchymal stem cells or osteoblasts are used per se or are transfected with bone growth factor genes selected from a group including bone morphogenetic protein (BMP) family genes or fibroblast growth factor family genes; for nerve repair neural cells and neural support cells are used per se or are transfected with genes selected from a group including nerve growth factor (NGF) gene or specific FGFs. The matrix is useful *inter alia* for the delivery of cells *in situ* to a specific site in the body, such as dopamine expressing cells to Parkinson's patients.

### Method of Matrix Preparation

Also disclosed is a method of preparing the porous, freeze-dried plasma protein matrix comprising plasma proteins and thrombin, having two opposing surfaces substantially parallel to the horizontal axis of the matrix and at least one additional surface extending along the periphery of the sponge substantially parallel to the vertical axis, wherein the plasma proteins crosslinked by the action of thrombin are present in a gradient having a higher concentration along one of the opposing surfaces and, wherein the average size of the pores in cross section is smaller along the surface of the matrix exposed to the higher concentration of thrombin comprising the following steps:
introducing a thrombin solution to a solid receptacle or mold;
layering a plasma protein solution over the thrombin solution in the solid receptacle or mold;
incubating under conditions appropriate to achieve clotting;
freezing the clotted mixture; and
lyophilizing the clotted mixture, to obtain a porous matrix.

The method may optionally further comprise the steps of
seeding the porous matrix with cells; and
implanting said cell-bearing porous matrix into an individual in need thereof.

The method may optionally further comprise
f) implanting the porous matrix per se into an individual in need thereof.

The porous plasma protein matrix may be prepared by transferring the thrombin solution into a mold or solid receptacle, carefully adding the plasma protein solution in order to minimize mixing of the two solutions, allowing the solutions to form a clot; freezing the clotted mixture and lyophilizing.

It is now disclosed that the properties of the matrix, including pore size, pore density, biodegradability may be controlled by adjusting the properties of the thrombin solution. The properties of the thrombin solution that may be varied include temperature, viscosity, volume, composition and concentration.

Without wishing to be bound by theory, a matrix formed using a chilled thrombin solution and or a chilled plasma protein solution undergoes cleavage and a crosslinking at a reduced rate. Alternatively the matrix may be cast using a thrombin solution comprising a viscosity-enhancing agent. Without wishing to be bound by theory, a viscosity-enhancing agent alters the diffusion capacity of the thrombin. Additionally the presence of certain viscosity-enhancing agents such as proteins, polypeptides and glycosaminoglycans may confer additional advantageous properties to the matrix, including improved cell adhesion, enhanced cell proliferation and or differentiation. In one embodiment the thrombin solution comprises at least one viscosity-enhancing agent. The thrombin solution may further comprise a bioactive agent.

The plasma protein solution may comprise fibrinogen and factor XIII. The plasma protein solution may consist essentially of fibrinogen; Factor XIII is introduced in the thrombin solution. The plasma proteins may be obtained from total blood, blood fractions, blood derivative, cryoprecipitate, recombinant proteins, plasma and plasma fractions. The plasma proteins may also be obtained from a commercially available source, including native or recombinant proteins. Preferably, the plasma protein solution is substantially devoid of organic chelating agents.

The plasma protein solution may be prepared so that the resulting matrix will comprise 10 mg plasma proteins/ml to 40 mg plasma proteins/ml, preferably 18 mg plasma proteins/ml to 30 mg plasma proteins/ml.

The plasma protein solution may further comprise at least one additive selected from the group consisting of calcium phosphate particles, glycosaminoglycans, polysaccharides, and synthetic polymers.

Preferably the at least one glycosaminoglycan is selected from crosslinked and non-crosslinked hyaluronic acid. The plasma protein solution may comprise hyaluronic acid and calcium phosphate particles.

The plasma protein solution may further comprise at least one bioactive agent selected from the group consisting of therapeutic proteins, platelets and platelet supernatant, analgesics, anti-microbial or anti-inflammatory agents and enzymes. The plasma proteins may further comprise one or more antifibrinolytic agents including aprotinin, tranexamic acid, epsilon-aminocaproic acid and alpha-2-macroglobulin.

The at least one bioactive agent is a therapeutic protein selected from the group consisting of growth factors and their variants. The growth factor may be selected from a fibroblast growth factor (FGF) and variants thereof. The FGF may be an FGF having the capacity to induce or enhance cartilage, bone or liver repair and regeneration and or angiogenesis. The growth factors may be incorporated at a wide range of concentrations, depending on the potency of the factor and the intended application.

The plasma protein solution may comprise hyaluronic acid and or heparin and a therapeutic protein selected from the FGF family of growth factors and variants thereof. Alternatively, the plasma protein solution comprises hyaluronic acid and or heparin and the thrombin solution comprises a therapeutic protein including an FGF. The bioactive agent such as a growth factor may be incorporated into the sponge *per se* or heparin bound. Heparin may be incorporated into the matrix to a final concentration of 0.01 ug/ml to 0.1 mg/ml. Preferably 0.1 ug/ml to 1.0 ug/ml. Crosslinked hyaluronic acid maybe incorporated into the matrix to a final concentration of 0.001% to 0.1%, more preferably 0.05% to 0.09%. Non-crosslinked hyaluronic acid may be incorporated into the matrix to a final concentration of 0.05% to 0.5%, more preferably 0.075% to 0.125%. In some embodiments both heparin and hyaluronic acid are incorporated into the matrix at respective concentration ranges. Preferably, the additive is incorporated into the matrix *ab initio.*

Surprisingly, in view of the known function of heparin as an anti-coagulant, the incorporation of heparin into the matrix does not interfere with either the formation of the matrix or the therapeutic benefits of the matrix. Without wishing to be bound by theory, heparin serves primarily to bind FGF or other therapeutic proteins and creates a depot for sustained release of said proteins. In addition, low molecular weight fragments of heparin released from the matrix may function as anti-inflammatory agents and assist in the healing process of diseased or traumatized tissue (US 5,474,987; 5,686,431; 5,908,837). The thrombin solution may be introduced into a solid receptacle or mold containing a membranous structure. The membranous structure may lie flat with respect to the solid receptacle or mold. The membranous structure may comprise a prefabricated porous or woven planar structure, preferably a matrix, sheet or a mat. The membranous structure may be selected from a natural or synthetic material. The planar structure may be a natural material comprising crosslinked collagen fibrils.

The method of preparing a plasma protein matrix may further comprise the steps of shaping the matrix for example by casting in a mold of desired shape, or by cutting or punching the matrix. The matrix may have any suitable geometric shape. The matrix may have a geometric shape adapted to fit a lesion, defect or void into which it is introduced. The lesion, defect or void may be present in any body tissue including skeletal tissue such as cartilage and bone, and soft tissue such as liver, pancreas, kidney, heart, bladder, breast.

The thrombin solution and or the plasma protein solution may further comprise particulate matter such as calcium salts including calcium phosphate particles, hydroxyapatite particles, bone chips or glass fibers that are able to impart advantageous properties to the matrix including strength, additional porosity or phasic release.

The sponge may further comprise at least one bioactive agent, added *ab initio* to either the thrombin solution or the plasma protein solution.

In its final form prior to use with cells the sponge is substantially dry and contains less than 15% residual moisture, more preferably less than 10% residual moisture.

Also disclosed are methods of treating an individual in need thereof. Also disclosed is the use of the fibrin matrix of the invention for treating injured or traumatized tissue, including cartilage and bone defects. The method of treatment described herein is advantageous in that it requires minimal preparation for use by the medical practitioner and provides a less traumatic surgical procedure for the patient..

The porous fibrin matrix may be used as a coating on synthetic or other implants such as pins and plates, for example, in hip replacement procedures. Thus, the present invention further provides implants or medical devices coated with the comprising the porous fibrin matrix of the invention.

Furthermore, the sponge may be used as a component of a two-phase or multi-phase material for tissue repair such as seen in osteochondral defects. In a non-limiting example, one layer may comprise a calcium phosphate material whilst an additional layer may comprise the sponge of the invention.

The plasma proteins may come from a commercial source, natural or recombinant proteins, or may be prepared from plasma. The plasma protein solution may derive from allogeneic plasma. A least one of the components, preferably the plasma proteins may be used for preparing the matrix derives from autologous plasma or recombinant proteins. All of the plasma components used in preparing the matrix may be autologous. A stable autologous thrombin component may be isolated from autologous plasma, according to methods known in the art for example those disclosed in US Patent No. 6,274,090 and Haisch et al (Med Biol Eng Comput 38:686-9, 2000). The plasma proteins may be isolated by a variety of methods, as known in the art and exemplified herein below, resulting in a fibrin matrix having substantially similar properties, as measured by pore size, elasticity, compression and cell bearing capabilities.

Blood may be drawn from a patient in need of tissue repair or regeneration, plasma proteins, are isolated from the autologous plasma and a matrix prepared therefrom. The platelets are optionally isolated and returned to the plasma.

A porous plasma protein sponge produced from a plasma protein solution is disclosed, wherein the fibrinogen solution is subjected to dialysis, preferably with a solution not requiring a complexing agent. While not wishing to be bound by any particular theory the substantial absence of organic complexing agents may provide the matrix of the present invention with properties beneficial to the proliferation and metabolism of certain cell types. As shown in the examples herein, the matrix of the present invention serves as an excellent support for chondrocytes and hepatocytes.

### Applications

The porous plasma protein matrix of the invention provides an unexpectedly advantageous support for cellular growth *in vitro* and *in vivo* and is useful as a scaffold for tissue engineering and repair applications. The present invention provides all components fundamental for tissue repair, thus facilitating the medical practitioner's task.

The *in vivo* uses of the plasma matrix are manifold. The matrix may be used as an implant per se, for providing mechanical support to a defective or injured site *in situ* and/or for providing a matrix within which cells proliferate and differentiate. The cells may be selected from stem cells or progenitor cells or from specialized cells such as chondrocytes, osteoblasts, hepatocytes, or mesenchymal, endothelial, epithelial, urothelial, endocrine, neuronal, pancreatic, renal or ocular cell types.

The matrix of the present invention can be utilized in reconstructive surgery methods for regenerating and/or repairing tissue that have been damaged for example by trauma, surgical procedures or disease. The present invention provides a matrix for use as an implantable scaffold per se for tissue regeneration. According to one embodiment of the invention, the matrix serves as both a physical support and an adhesive substrate for *in vivo* cell growth. As the cell populations grow and the cells function normally, they begin to secrete their own extracellular matrix (ECM) support. The scaffold polymer is selected to degrade as the need for an artificial support diminishes.

Scaffold applications include the regeneration of tissues such as neuronal, musculoskeletal, cartilaginous, tendonous, hepatic, pancreatic, renal, ocular, arteriovenous, mammary, urinary or any other tissue forming solid or hollow body organs. In orthopedic applications, the matrix may be used *per se* or in combination with other therapeutic procedures including chondral shaving, laser or abrasion chondroplasty, and drilling or microfracture techniques. Some typical orthopedic applications include joint resurfacing, meniscus repair, non-union fracture repair, craniofacial reconstruction or repair of an invertebral disc.

The matrix of the invention is useful, *inter alia*, as an unexpectedly advantageous support for *in vitro* cellular growth. In a certain embodiments of the present invention cells may be cultured on the matrix for subsequent implantation or other laboratory or biomedical applications. Stem cells derived from any tissue or induced to differentiate into a specific tissue type may be utilized. Preferably the cells are derived from autologous tissue. For example, for culturing cartilage, chondrocytes or mesenchymal stem cells may be seeded on the matrix. In specific embodiments of the invention, chondrocytes or chondrocyte progenitor cells can be seeded on the matrix prior to implantation or at the site of implantation. Another *in vitro* use includes a depot for bioactive agents in cell, tissue or explant culture.

The matrix of the present invention may be used as a support for chondrocyte growth and as a scaffold for neo cartilage formation. However, the plasma protein matrix of the invention may be used as a surface useful for tissue culture for any suitable cells, such as mesenchymal cells or other tissue forming cells at different levels of potency. For example, stem cells, mesenchymal stem cells, progenitor cells can be seeded on the matrix of the invention. A lineage-committed progenitor cell is generally considered to be capable of a limited number of mitotic divisions and will eventually differentiate into a specific cell type.

A person skilled in the art can adjust the procedures exemplified below in accordance with specific tissue requirements. Preferably, the matrix of the present invention is implanted per se, and serves as a scaffold for cellular growth *in situ.* The matrix may be seeded with cells, such as cells that have been expanded *in vitro,* and implanted. Alternatively, the matrix may be seeded with cells, left to incubate and the sponge comprising the cells implanted at a site in need of tissue repair or regeneration. In certain applications more than one matrix may be implanted at a particular site.

In the reconstruction of structural tissues like cartilage and bone, tissue shape is integral to function, requiring the molding of the matrix into three dimensional configuration articles of varying thickness and shape. Accordingly, the matrix of the invention may be formed to assume a specific shape including a sphere, cube, rod, tube or a sheet. The shape may be determined by the shape of a mold, receptacle or support which may be made of any inert material and may be in contact with the matrix on all sides or on a limited number of sides. The matrix may be shaped in the form of body organs or parts and constitute prostheses. Removing portions of the matrix with scissors, a scalpel, punch, a laser beam or any other cutting or shaping instrument can create any refinements required in the three-dimensional structure.

The methods for seeding cells on the matrix are manifold. In a non-limiting example, the cells may be seeded with the desired cells by any method of seeding including surface seeding, spray seeding or absorption.

Furthermore, the sponge may be used as a component of a two-phase or multi-phase material for tissue repair such as seen in osteochondral defects. In a non-limiting example, one layer may comprise a calcium phosphate material whilst an additional layer may comprise the sponge of the invention. Gao et al. (Tissue Engin. 8:827-837, 2002) describe a repair method for osteochondral defects using a composite material comprising an injectable calcium phosphate and a hyaluronic acid sponge.

The following examples are intended to be merely illustrative in nature and to be construed in a non-limitative fashion.

### EXAMPLES

### Example 1: Preparation of a Plasma Protein Matrix

It is to be understood that many different methods of preparing clottable plasma proteins (CPP=clottable plasma proteins) are known in the art and are useful in the preparation of the matrix of the present invention. The major clottable plasma protein is fibrinogen. A non-limiting example of a protocol for the preparation of a fibrinogen-enriched plasma protein solution is taught in Sims, et al. (Plastic & Recon. Surg. 101:1580-85, 1998). Any source of plasma proteins may be used.

### Materials and Methods:

Source of plasma proteins e.g. Quixil (Omrix, IL), Beriplast (Aventis, DE), allogeneic or autologous blood plasma (Stock solution about 40 mg/ml to about 80 mg/ml) or purified fibrinogen (about 67 mg/ml.)
Factor XIII: 60 IU/ml stock solution
Calcium Chloride: about 5 mM
Thrombin: (human, stock solution: 1000 International Units/ml, Omrix, IL or 1,500 IU/ml from lyophilized, Aventis DE)
Hyaluronic acid (HyA); crosslinked (Hylan (Synvisc), approx. MW 6 x 10⁶, Genzyme, USA) or non-crosslinked (approx. MW 8 x 10⁵, MTF, USA; approx. MW 3.6 x 10⁶, BTG, IL)
Fibronectin (1 mg/ml) was added to the thrombin solution to a final volume of 10%, 25% and 50%. The matrices were prepared as described.
Collagen (Type I, 10 mg/ml) was added to the thrombin solution to a final volume of about 10%, 25% and 50%. The matrices were cast as described below.
Calcium phosphate: Calcium phosphate particles (about 2-4 µm diameter particles) were added to 100 µl thrombin and the matrix cast as described below.

Certain examples of matrix components are shown below in the table 1 below.

| Clottable plasma protein mg/ml | % HyA plasma protein solution | Thrombin Conc (IU/ml) | Ratio plasma proteins: thrombin (v/v) | %HyA in thrombin solution | Clotting temp. (°C) | Clotting Time (hours) |
|---|---|---|---|---|---|---|
| 20 | 0.075 | 600 | 50:1 | 0 | 17 | 3 |
| 20 | 0.075 | 600 | 30:1 | 0 | 17 | 3 |
| 20 | 0.075 | 600 | 15:1 | 0 | 17 | 3 |
| 24 | 0.1 | 720 | 42:1 | 0 | 17 | 6 |
| 24 | 0.1 | 720 | 30:1 | 0.01 | 17 | 10 |
| 24 | 0.1 | 720 | 30:1 | 0.02 | 17 | 10 |
| 24 | 0.1 | 720 | 30:1 | 0.015 | 17 | 10 |
| 24 | 0.1 | 864 | 30:1 | 0 | 17 | 10 |
| 24 | 0.1 | 864 | 30:1 | 0.01 | 17 | 10 |
| 24 | 0.1 | 864 | 30:1 | 0.01 | 4 | 10 |
| 24 | 0.1 | 864 | 30:1 | 0.03 | 17 | 4, 6, 10 |
| 24 | 0.1 | 864 | 12:1 | 0.02 | 17,22 | 4, 6 |
| 24 | 0.1 | 720 | 12:1 | 0.01 | 17,22 | 4, 6 |
| 24 | 0.1 | 720 | 5:1 | 0 | 17 | 3, 6, 10 |
| 25 | 0.1 | 375 | 30:1 | 0.01 | 17 | 10, 24 |
| 25 | 0.1 | 750 | 30:1 | 0.01 | 37 | 2, 3 |
| 25 | 0.1 | 750 | 30:1 | 0.01 | 17 | 6, 10 |
| 25 | 0.1 | 750 | 30:1 | 0 | 17 | 3 |
| 25 | 0.1 | 750 | 10:1 | 0.01 | 37 | 3 |
| 25 | 0.1 | 1500 | 42:1 | 0.01 | 17 | 6, 10 |
| 25 | 0.1 | 1500 | 30:1 | 0.01 | 17 | 10 |
| 27 | 0.1 | 810 | 30:1 | 0.1 | 17 | 10 |

Typically, the "diffusion" sponge was formed in the following manner:

The mold, a solid receptacle or well, was coated with hyaluronic acid (0.01 %). The thrombin solution was introduced into the mold. In certain tests the thrombin solution further comprised a viscosity-enhancing material such as collagen, hyaluronic acid, fibronectin, glycerol or other natural or synthetic materials. The plasma protein solution was dispensed over the thrombin solution in the mold; care was taken to prevent mixing of the two solutions. The liquid phases were allowed to incubate at least until a clot formed. Incubation typically continued for several hours, as shown in Table 1. Clots were allowed to form at about 17°C to about 37°C. The clot was frozen for about 1 hour at about -20°C, -40 °C or at about -70°C and lyophilized. Exemplary 10 mm matrices wre formed by dispensing about 10 ul to about 60 ul thrombin solution and layering with about 300 ul of a plasma protein solution. Exemplary 35 mm matrices were formed by dispensing about 70 ul to about 600 ul thrombin solution and layering with about 3 ml of a plasma protein solution. In certain examples the sponges were seeded with cells including chondrocytes, hepatocytes and other types, see Example 4.

A sponge of any size and shape may be cast. A 35 mm diameter sponge is particularly useful for the treatment of larger lesions, such as those that may develop in osteoarthritis. The matrix may be shaped either before or following cell seeding. In the laboratory, sterile plates having 6, 12, 48 or 96 wells were typically used for casting.

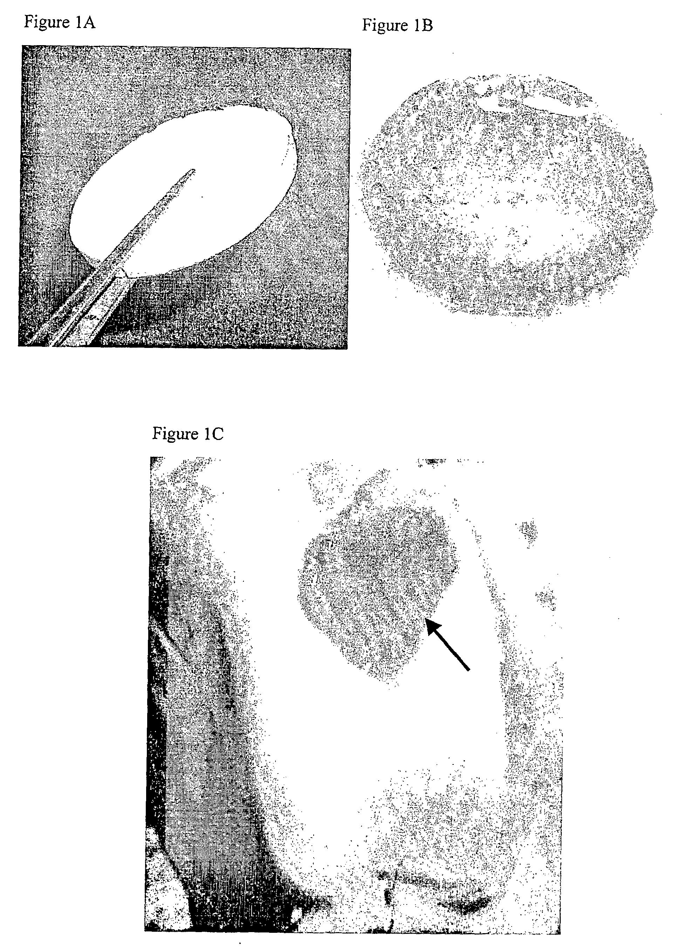

An example of a dry 35 mm diameter sponge is shown in Figure 1A (3 ml CPP solution: 24 mg/ml CPP, 0.1% HyA; 72 µl thrombin solution: 720 IU/ml, 0.01% HyA). A photograph of a cell-bearing sponge is shown in Figure 1B and the cell-bearing sponge implanted into a lesion introduced into the articular surface of a pig's knee is shown in Figure 1C.

Matrices prepared from purified fibrinogen, were cast in the following manner:

A fibrinogen stock solution was prepared by mixing 432 µl purified fibrinogen, 120 µl hyaluronic acid and 650 µl purified distilled water. Matrices comprising different concentration s of Factor XIII were prepared. Either 10 ul of a thrombin solution comprising thrombin (700 IU/ml), and Factor XIII (1 IU) or 20 ul of a thrombin solution comprising thrombin (350 IU/ml) and Factor XIII (4 IU) were dispensed into 10 mm hyaluronic acid-coated wells. About 300 µl of the fibrinogen stock solution (24 mg/ml + 0.1% HyA) was dispensed over the thrombin solution and a clot was allowed to set at room temperature for about 8 hours. The clot was frozen for 1 hour at -40°C, lyophilized for about 12 hours and seeded with chondrocytes.

The composition of the thrombin solution was varied to produce plasma protein matrices having additional advantageous properties, including enhanced cell attachment, cell proliferation and differentiation. The parameters that may be varied include thrombin concentration, viscosity, volume, temperature and composition. For example, the presence of certain proteins or other viscosity-enhancing agents were added. In all cases the thrombin solution and the plasma protein solutions were cast sequentially, either solution may be cast first. A thrombin solution of about 300 IU/ml about 1500 IU/ml yielded a sponge with good physical and biological properties.

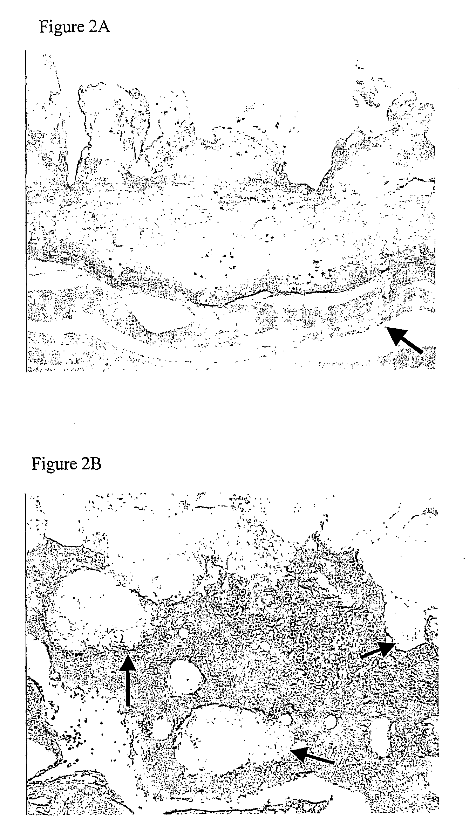

Collagen membrane: In certain experiments a collagen membrane (about 0.1 mm thick) comprising crosslinked collagen fibrils was cut to fit a 10 mm well and placed in the bottom of the well. The membrane was impregnated with thrombin solution (864 IU thrombin/ml) and a plasma protein solution (20 mg/ml plasma protein + 0.075% hyaluronic acid) was dispensed carefully over the thrombin solution. The clot set for 40 minutes and was frozen and lyophilized. Figure 2A shows a histological cross section of a matrix prepared layered upon a collagen membrane. The arrow shows the collagen membrane layer.

In other examples a matrix comprising calcium phosphate particles was prepared. A thrombin solution (864 IU/ml + 1 gm calcium phosphate particles) was introduced into a 10 mm well. Plasma protein solution (20 mg/ml plasma protein + 0.075% hyaluronic acid) was dispensed carefully over the thrombin solution. The clot set for 40 minutes and was frozen and lyophilized. Figure 2B shows a cross section of a matrix comprising the calcium phosphate particles. The arrows mark the particles.

In some examples, the plasma protein solution (CPP=clottable plasma proteins) and the thrombin solution were cast in a 35 mm diameter well (for example 3000 µl CPP and about 100 µl or about 70 µl thrombin solution). A 6 well ELISA plate was coated with 0.01 % hyaluronic acid and was left to dry. In another example, a 48 well ELISA plate (∼10 mm diameter wells) was used and about 20 ul or about 50 µl thrombin solution was added to the wells followed by the addition of about 500 ul CPP. The 35 mm diameter clot was formed by leaving the mixture to polymerize at room temperature (∼25°C) for about 1 hour to about 24 hours, followed by freezing, lyophilizing and drying. The matrices were lyophilized at about -70°C to about -20°C overnight (10-16 hours) followed by raising the temperature to 20°C over a period of about an hour prior to releasing the vacuum. The matrices were prepared under sterile conditions.

In some examples, the matrices were prepared using a plasma protein solution comprising certain additives including disaccharides, polysaccharides, GAGS and synthetic polymers. All additives were filtered (0.2 µm) and were added to the plasma protein solution. When hyaluronic acid was incorporated in the matrix, the plasma protein solution and hyaluronic acid solution were incubated together before casting.

In another example, the lyophilized matrix was seeded with cells and a thrombin solution (750 IU/ml) was dispensed onto the surface of the matrix exposed to the lower concentration of thrombin, and allowed to clot. Without wishing to be bound by theory, the additional thrombin interacts with the fibrinogen/fibrin dimers at the surface of then matrix and undergoes clotting to further strengthen the matrix.

For comparison purposes, "mixed" matrices were prepared by premixing the thrombin and plasma protein solutions before casting, freezing and lyophilizing. "Nonmixed" matrices were prepared by dispensing 1 ml of a thrombin solution into a 35 mm well and dispensing 2 ml plasma protein solution over the thrombin solution. A clot was allowed to set and the clot was freeze-dried.

### Example 2: Isolation of Partially Purified Plasma Proteins from Whole Plasma

Plasma protein may be prepared from different sources such as fresh plasma, fresh frozen plasma, recombinant proteins and xenogeneic, allogeneic or autologous blood. The fresh frozen plasma may be received from any blood bank or directly from an individual who is to undergo implantation of the matrix (autologous blood plasma) and processed according to the protocol presented in WO 03/007873. Plasminogen-free plasma protein solutions may be prepared according to methods known in the art, including methods taught in PCT patent publications WO 02/095019 and WO 95/25748.

The plasma protein matrices may further comprise endogenous or exogenous blood platelets or platelet supernatant. In a non-limiting example, a method for the isolation of a platelet-enriched plasma is taught in US 6,475,175. Platelet supernatant is made by exposing isolated platelets (obtained from the Israel blood bank) to thrombin as described (Gruber et al., Clin Oral Implants Res 13:529-535, 2002), collecting the supernatant and adding it to the plasma protein solution prior to sponge formation.

### Example 3: Physical and Mechanical Properties of Matrix

In general, matrices for tissue engineering are characterized according to several criteria, including chemical nature, homogeneity, porosity, adhesion, biocompatibility and elasticity, amongst others (Hunziker, Osteoart. Cart., 10:432-465, 2002). Table II in that reference lists several of the properties and the biological basis of these properties.

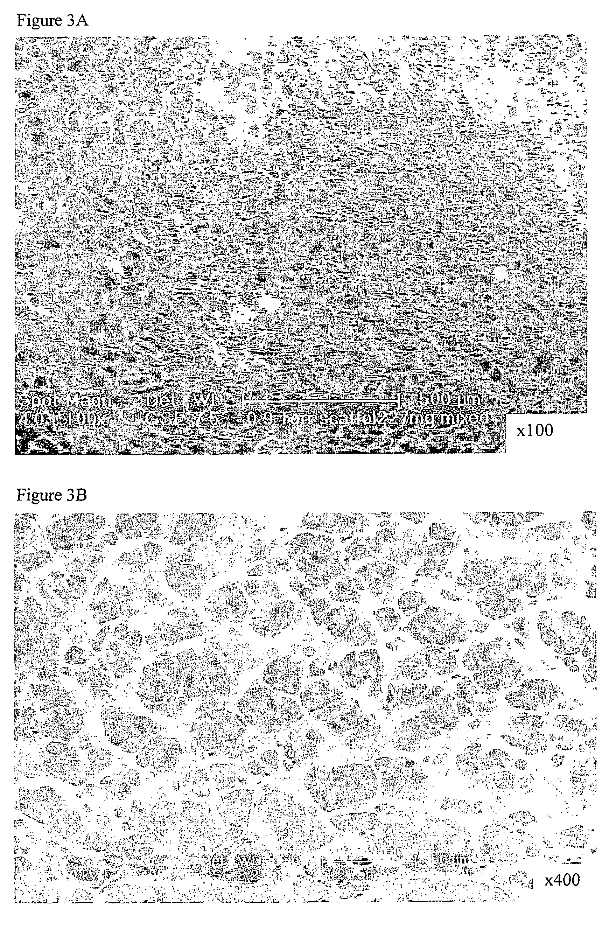

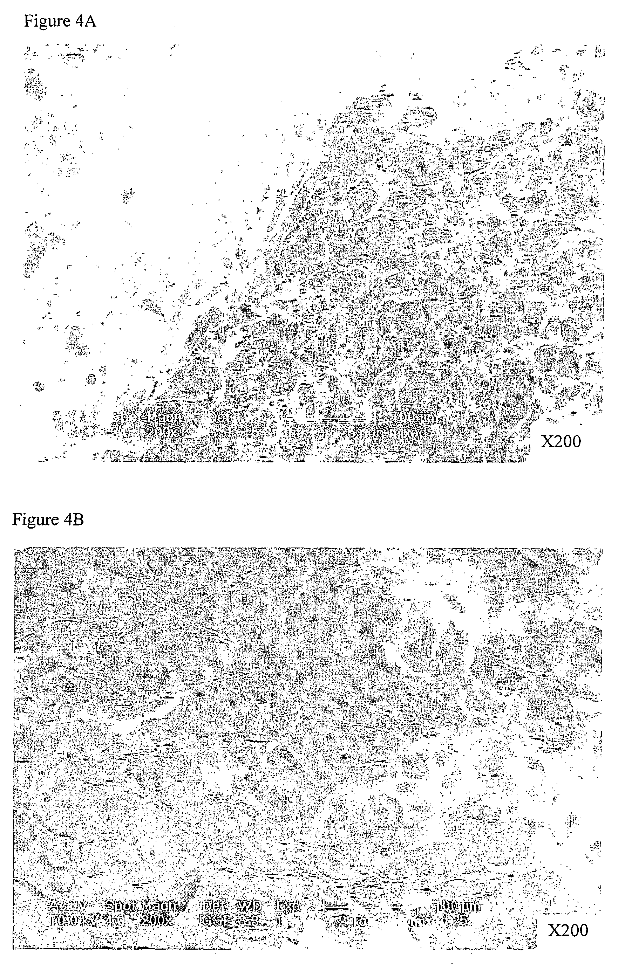

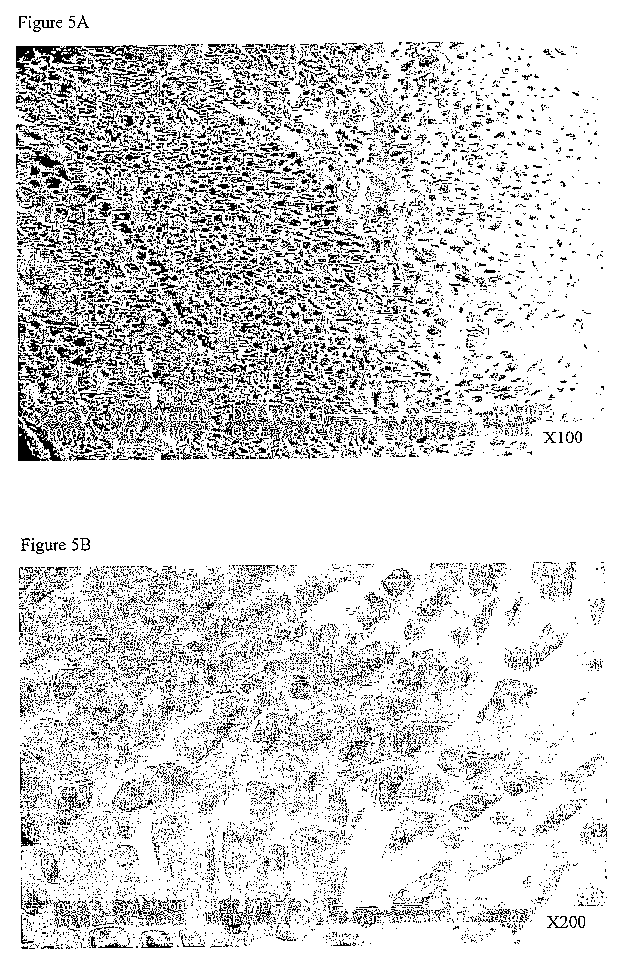

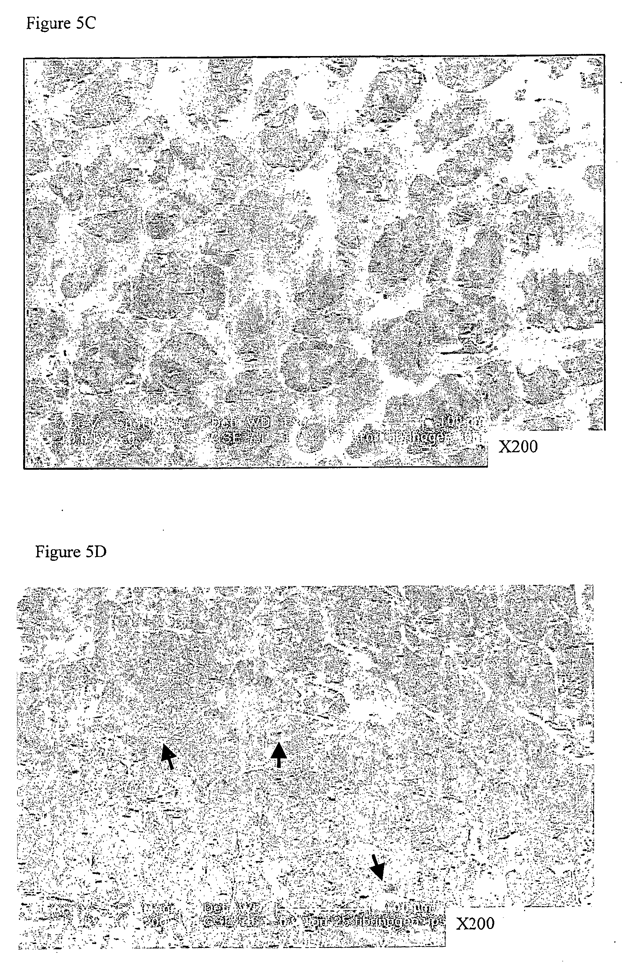

Several of the aforementioned properties have been and will be determined for the matrices of the present invention. Scanning Electron Microscope (SEM) analysis was performed in order to study the ultra structure of the matrices. Comparisons were made between three different types of matrices 1) a "mixed" matrix wherein the thrombin and plasma proteins are mixed prior to casting; 2) a "nonmixed" matrix where the thrombin and plasma proteins are dispensed sequentially and are present in a ratio of about 1:2, respectively; 3) a "diffusion matrix" wherein the thrombin having a thrombin diffusion gradient. Figures 3A and 3B show SEM photos of the upper surface (top) of a premixed matrix (25 mg/ml CPP, 0.1% HyA, 750 IU/ml thrombin). Figures 4A and 4B show SEM photos of the "nonmixed" matrices described above. Figure 4A is a photo of the periphery of the matrix while Figure 3B is photo of the center upper surface of the matrix. Figures 5A and 5B show SEM photo of a surface of the matrix having a lower thrombin concentration (side for cell seeding) in a matrix that was prepared by allowing the clot to set for about 3 hours while Figure 5C shows the surface of the matrix having a lower thrombin concentration in a matrix that was prepared by allowing the clot to set for about 10 hours. Figure 5D shows the surface of the matrix having a higher thrombin concentration (3 hour clot setting).

The number of pores per 500 µm² and the average size of the pores (channel openings) were determined by measuring perpedicular axes of the pores in cross section from the SEM photos. Values are presented in Table 2, below.

The values are presented as d1 x d2, where d1 is one axis and d2 is the axis perpendicular to d1. Comparisons were made between a ``nonmixed" matrix having a ratio of plasma proteins to thrombin of about 2:1 (v/v) and a diffusion directed matrix having a ratio of plasma proteins to thrombin of about 30:1 (v/v). Both matrices were prepared from plasma proteins (20 or 25 mg/ml with 0.1% hyaluronic acid) and thrombin (about 750 IU /ml) and were prepared by casting the thrombin solution into a mold followed by casting of the plasma protein solution over the thrombin.

**Table 2: Pore number and pore size in different matrices**

| | 25 mg/ml nonmixed 3 hr* -70°C "Top" | 25 mg/ml diffusion 3h* -70°C "Top" | 25 mg/ml diffusion 3h* -70°C "bottom" | 24 mg/ml diffusion 10h* -40°C "Top" | 24 mg/ml diffusion 10h* -40°C "bottom" | 24 mg/ml diffusion 10h* -70°C "Top" | 24 mg/ml diffusion+ 10h* -70°C "bottom" |
|---|---|---|---|---|---|---|---|
| Avr. no. pores (per 500 µm²) | 15 | 53 | 21 | 44 | 26 | 49 | 33 |
| Average pore size (µm) | 22x22 | 32x40 | 10x9 | 33x34 | 14x16 | 21x22 | 9x9 |

| | | | | | | | |
|---|---|---|---|---|---|---|---|
| * number of hours clot allowed to set before freeze-drying + thrombin solution comprises albumin, collagen and elastin temperature refers to freeze-drying temperature non-mixed refers to a matrix as defined and described in Example 1a. Diffusion refers to the matrix of the invention prepared as described in Example 1. "top" refers to the surface of the sponge having a lower thrombin concentration (the side used for cell seeding) while "bottom" refers to the opposing surface of the sponge having a higher thrombin concentration. | | | | | | | |

The present invention provides a matrix having a large number of channel openings in addition to pores having a larger size. These characteristics allow for better distribution and dispersion of the cells throughout the matrix and are beneficial for cell attachment, proliferation and or differentiation.

Mechanical property measurements are performed, for example, using a Chatillon TCD200 machine with a digital force gauge DF12. Each plasma protein sponge is approximately 2.5 cm long, 0.5 cm wide; and is fully lyophilized. Deformation represents the elasticity of the sponge, i.e. the amount of pull as measured in millimeters (mm) that may be exerted until the sponge tears. Force is calculated in kiloPascal (kPa) and represents the amount of energy required to tear the sponge strips. The thickness of the sponge is taken into consideration when making the calculation.

The amount of residual moisture in the matrix is determined using a variation of Baker's technique. A matrix made with 100µl of thrombin solution (720 IU/ml) and 3 ml plasma protein solution (24 mg/ml + 0.1 % HA) was weighed immediately after lyophilization and then every twenty minutes for 2 hours. The matrix was oven dried for 18 hours and the weighing procedure repeated. The weight increase as a function of time (= moisture content increase) after lyophilization and after drying in a 105°C oven was determined and plotted. The residual moisture of the lyophilized matrix at time zero was extrapolated from the graph, at the intersection of the slope with the y axis. The residual moisture was calculated as follows: (0.1698-0.1617)* 100/0.1698= 4.8%.

In its final form, prior to use with cells, the sponge is substantially dry and contains less than 10% residual moisture, more preferably less than 5% residual moisture.

### Example 4: Cell Seeding on the Matrix

Different methods of seeding cells onto the sponge may be used. Important to seeding is cell adherence, migratory capacity and proliferation of cells within the matrix. Cells may be suspended in medium, PBS, or any compatible buffer alone or in the presence of serum and or bioactive agents. Cells may be seeded by placing a drop of liquid containing cells on the sponge and allowing the cells to adsorb into the sponge. Alternatively, the cells in the liquid may be absorbed into the sponge by placing the sponge in a container holding a suspension of cells. Other methods including spray seeding have also been shown to be effective.

One particular advantage of the matrix of the present invention is the high level of cell viability and excellent cell distribution throughout the matrix.

### Materials and Methods:

Matrices comprising different concentrations of plasma proteins and thrombin were tested. Varying numbers of cells were seeded on the sponge. In one assay, 7x10⁶ chondrocytes were spray seeded on 10 mm diameter matrices (24 mg/ml CPP, 0.1% HyA, thrombin solution (about 720 IU /ml) and viscosity-enhancing agents).

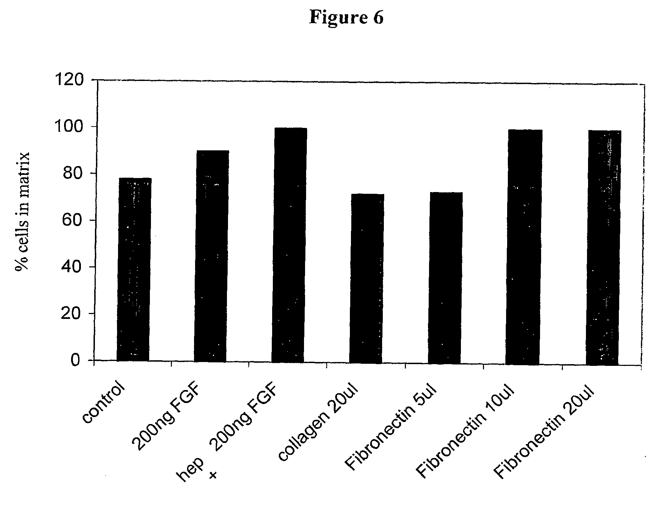

Following a three-day incubation for the seeded matrices, some of the matrices were collagenase degraded and cells counted following trypan blue staining. The graph in Figure 6 shows the percentage of live cells remaining in the different matrices of the present invention following three-day incubation. Control refers to the matrix *per se* devoid of agents in the thrombin. 200ng FGF refers to 200 ng/ml FGF2 that was added to the thrombin solution before formation of the matrix. Hep + 200 ng FGF refers to heparin (200ng/ml) present in the plasma protein solution and 200ng/ml FGF present in the thrombin solution prior. The other columns refer to the amounts of collagen I and fibronectin present in the thrombin solution. The number of live cells remaining in the matrices is overall very high (> 70%).

Samples of the cell-bearing sponges or matrices, were paraffin-embedded and sections prepared using a microtome. The histological sections are further stained using different biological stains including hematoxylin and eosin (H&E), toluidine blue and fast red, Masson's trichrome stain and others. All sponges exhibited similar cell distribution, with live cells present throughout all layers of the sponge.

### Example 5: in vitro Degradation Assay

The *in vitro* degradation assay is carried out to determine the rate of degradation of the sponge. Without wishing to bound to theory, the greater the extent of cross linking in a fibrin matrix the slower its degradation rate. The assay is performed in the following manner:

Five sponges prepared in 96 well plates (5 mm diameter) are placed in 48 well plates and 750 ul of 10M urea was added to cover the sponges. Samples of 20 ul are collected from each well at the following points: 1, 2, 3, 4, 5, 8 minutes, 10 minutes, 30 minutes, 1 hrs. Protein from each sample is measured in a standard Bradford assay.

### Example 6: Release of Bioactive Agents from the Matrix

For certain applications, sustained release of a bioactive agent such as a growth factor may be desirable. The incorporation and release of growth factors from the matrix of the invention was assessed *in vitro* and may be assessed *in vivo* using radiolabeled or tagged growth factors, for example fluorescent-labeled, alkaline phosphatase labeled or horseradish peroxidase-labeled growth factor. The fraction and rate of released agent is measured by following the radioactivity, fluorescence, enzymatic activity or other attributes of the tag. Similarly, sustained release of enzymes from the matrix may be determined by analyzing enzymatic activity into the microenvironment in an *in vitro* or *in vivo* assay.

For example, sponges comprising a heparin binding growth factor such as FGF2 may be prepared in one of several ways: FGF2 is bound to heparin and the mixture is added to either the thrombin solution or to the plasma protein solution *ab initio.* In another non-limiting example, each component (heparin and a heparin binding growth factor) is added separately to the individual solutions: for example heparin is added to the plasma protein solution while FGF2 is added to the thrombin solution. Sponges are cast and FGF2 release is determined in an FDCP assay, *vide supra.*

FDCP Assay: The FDCP cell line is a murine immortalized, interleukin 3-dependent cell line of myelocytic bone marrow origin that does not express endogenous FGF Receptors (FGFR). Upon transfection with FGFR (FGFR1-4) cDNA, the FDCP cell line exhibited a dose-dependent proliferative response to FGF that can replace the dependence on IL-3. FGFR transfected FDCP cells can therefore been used to screen for FGFR signaling. FDCP cells response to various ligands is quantitated by a cell proliferation assay with XTT reagent (Cell Proliferation Kit, Biological Industries Co.). The assay measures the capability of mitochondrial enzymes to reduce tetrazolium salts into a colorigenic compound, which can be quantitated and is indicative of cell viability.

### Example 7: Chondrocyte Isolation and Culturing

Reagents:
Collagenase Type 2; Worthington Biochemical Corp. (Cat. #: 4147)
Stock solution: 1700 units/ml in medium (in MEM)
Dulbecco's MEM (DMEM) (Gibco BRL, cat. no. 41965)
Minimal Essential Medium (MEM) Gibco BRL (cat: 21090-022)
Fetal Bovine Serum (FBS); Gibco BRL (cat: 16000-044)
L- Glutamine Solution; Gibco BRL (cat: 25030-024)
Complete medium: MEM supplemented with 10% fetal calf serum (FCS), 2 mM L-Glutamine and 100U/ml penicillin, 100µg/ml streptomycin
Preparation of Implants for Treatment of Articular Cartilage Defects

The sponge of the present invention may be used as a cell bearing scaffold for tissue repair and regeneration. In one embodiment, cells are cultured on the sponge *in vitro,* prior to implantation. In another embodiment, the sponge is seeded with cells before implantation. In yet another embodiment the matrix is implanted and cells are seeded on or in the vicinity of the matrix, *in situ.*

Biopsies from human or porcine articular cartilage were wiped with 70% alcohol, and placed in laminar flow hood. The tissue was diced to approximately 1-2 mm pieces, washed aseptically with PBS and placed in a new tube containing 2 ml DMEM medium and 2 ml collagenase solution. The mixture was shaken gently in a 37 °C incubator over night. When most of the sample was digested, the suspension was poured through sterile gauze to remove matrix debris and undigested material. The filtrate was centrifuged and washed twice in DMEM to remove residual enzyme.

The number of cells was determined by a hemocytometer and viability was determined by Trypan blue exclusion. The cells were plated in 25 cm² or 75 cm² tissue culture flasks in human or fetal calf serum and culture medium at a concentration of about 3x10⁵ cells/ml (25 cm² flask) or 1x10⁶ cells/ml (75 cm² flasks). Flasks were incubated at 37°C, 5% CO₂ atmosphere and 95% humidity. The medium was changed every three to four days. The cells reached confluency after about one week incubation.

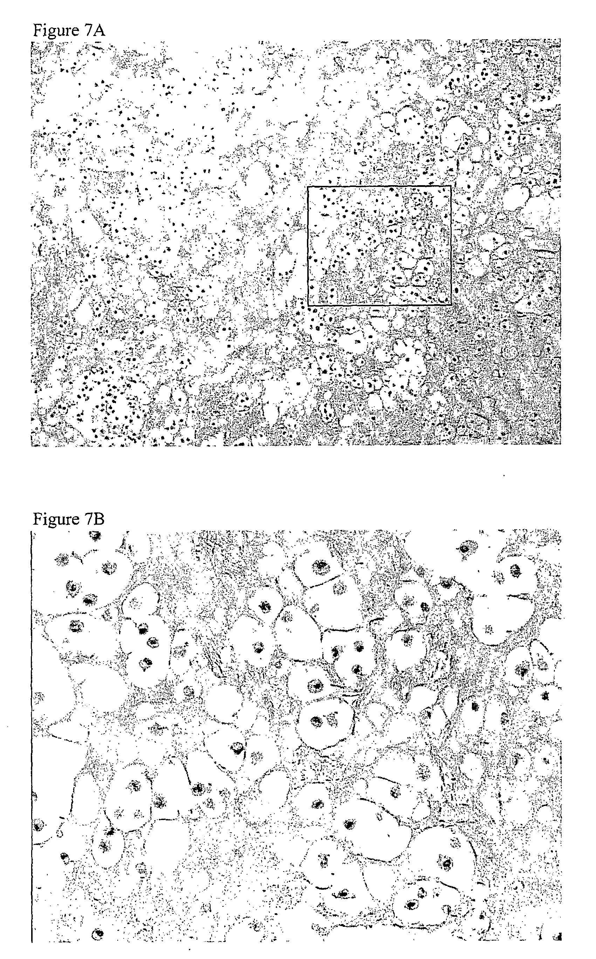

At confluency, the cell medium was replaced with 3 ml of a trypsin-EDTA solution. Thirty ml MEM+ FBS was added, the solution was centrifuged at 800g for 10 minutes. The pellet was gently dispersed and the cells were counted. To create a cell-bearing matrix, 10² -10⁶ cells were seeded on a fibrin scaffold of 9 mm in diameter and a thickness of 2 mm (approximately 0.2 cm³) or 35 mm diameter and about 2.5 mm thick (∼1.2 cm³). The matrices were incubated at 37 °C for about 1 hour and 1 ml of fresh medium was added to each. The medium was replaced with fresh medium and the matrices were incubated for several days to several weeks before being analyzed for cell proliferation, cell differentiation and histology. Figures 7A and 7B show histological scrossections of chondrocyte-bearing matrices.

The cells grown on the matrices express chondrocyte differentiation markers including glycosaminoglycan (GAG) production. GAGs may be identified by staining tissue or matrix sections with Alcian blue and quantitated using the DMB Dye (3,3'-dimethoxybenzidine dihydrochloride) method. Extracellular matrix proteins may also be identified by staining with toluidine blue and fast red or by RT-PCR analysis of RNA.

In another examples, spinal disc cartilage was biopsied and the cells cultured for implantation on a matrix.

### Example 8: Hepatocyte Culturing

To determine the capacity of the plasma protein matrix to support cell growth for tissue regeneration and repair, primary rat liver hepatocytes were cultured on the matrix.

Primary rat hepatocytes were isolated by perfusing the rat liver. Briefly, a veinflon was inserted in the portal vein of laparotomized rats anesthetized with Nembutal. The liver was then perfused with a 5mM EGTA solution in Lefferts buffer (10 mM Hepes, 3 mM KCl, 130 mM NaCl, 1 mM NaH₂PO₄.-H₂O, 10 mM D-Glucose, pH 7.2) using a peristaltic pump at a speed of 12 ml/min for a total of 4 minutes. The liver was washed for 2 minutes with Lefferts buffer, followed by 15 minutes perfusion with 0.35 mg/ml collagenase Type 1 (333 U/mg, Worthington Biochemical Corporation, NJ, USA) in Lefferts buffer with 0.027% CaCl₂. The liver was gently placed in the HDM medium described below and hepatocytes were released from the Glisson capsule using two scalpel blades. The cells were washed by centrifugation at 50g twice and suspended at the desired cell concentration.

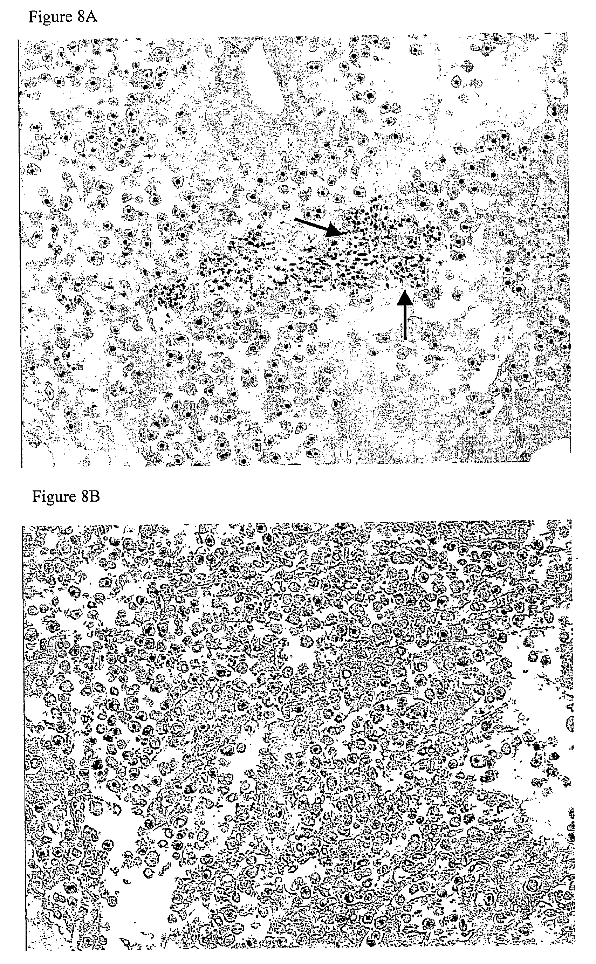

Ten mm or 35mm diameter sponges comprising plasma proteins (24 mg protein/ml), 0.1% hyaluronic acid and 720 IU thrombin/ml were prepared. Approximately 6.6 x10⁵ primary hepatocytes were seeded on the sponges in HDM (hormonally defined medium) without serum and allowed to incubate for three days at which histological samples were made and stained with H&E. Figure 8A shows a representative section of a sponge comprising hepatocytes, following a three day incubation. Note the good dispersion of cells throughout the matrix and the presence of cells maintaining their hepatic characteristics. The arrows denote bile duct cells. Figure 8B shows the section of a matrix that had been seeded with hepatocytes and incubated 14 days. The cells retain good morphology as the matrix begins to degrade.

### Example 9: Cell Proliferation Assay

Proliferation of the cartilage cells on the matrix of the invention was quantitated by one of two methods, CyQUANT® (Molecular Probes) or XTT reagent (Biological Industries, Co.). The matrix was dissolved in collagenase or other enzymes and the cells collected by centrifugation and analyzed according to directions provided by the manufacturers.

### Example 10: Ectopic Cartilage Formation in Nude Mice

The assay is designed to determine the ability of isolated chondrocytes to create neocartilage in an ectopic site, and to determine the quality of this cartilage compared to natural cartilage. Human and porcine chondrocytes seeded on a matrices of the invention are used to induce ectopic cartilage on the backs of nude mice

Treatment arms: The study groups included different amounts of cells seeded onto the plasma protein matrix substantially devoid of plasminogen. Human or porcine chondrocytes were seeded onto a plasma protein sponge from a 96 well plate (∼65 ul). The control group consisted of matrices implanted without cells.

Seeding: Sponges were seeded with human or porcine chondrocytes (10⁵ - 10⁶/ 20 ul culture medium in a 96 well plate and incubated at 37°C for 1 hour. Culture medium was added to the well and the sponge incubated 24-48 hours. The sponge was placed into subcutaneous incisions made on the back of nude mice.

Implantation procedure: Animals are anesthetized using ketamine-xylazine. Back skin is shaven and cleaned using alcohol. Two incisions, are made on each side of the back, parallel to the spine. A subcutaneous pocket or a pocket in the muscle fascia is made from each incision using blunt dissection. The sponges are implanted in the pockets according to treatment arms and the skin is closed with single suture. Each treatment is repeated 5 times and each mouse is implanted with 4 sponges.

The treatment arms are presented in table 3 herein below.

**Table 3: Ectopic Cartilage Experimental Setup**

| Mouse No. | Left proximal | Left distal | Right proximal | Right distal | Tagging |
|---|---|---|---|---|---|
| 1 | 10⁵ Human | 10⁶ Human | 10⁵Porcine | 10⁶ Porcine | No tag |
| 2) | 10⁶ Human | 10⁵ Human | 1x10^6 Porcine | w/o cells | 1 Rt ear |
| 3 | 10⁶ Porcine | 10⁵ Porcine | 10⁵ Human | 10⁶ Human | 1 Lt ear |
| 4 | 10⁵ Porcine | w/o cells | 1x10^6 Human | 10⁵ Human | 2 Rt ear |
| 5 | 10⁶ Human | 10⁶ Porcine | Sponge w/o cells | 10⁵ Human | 2 Lt ear |
| 6 | w/o cells | w/o cells | 10⁶ Porcine | 10⁵ Porcine | RT+LT |

Induced cartilage formation evaluation: One or four weeks post implantation the mice are sacrificed and the implants with their surrounding tissue retrieved and prepared for histology evaluation. The microscopically assessment consists of a complete morphological description of the implant. Additional analysis include H&E staining safranin O, alcian blue and anti-collagen type II staining.

### Example 11: Sheep Model of Cartilage Repair

This study was designed to evaluate the capacity of the chondrocyte embedded matrix of the invention to repair cartilage in a large animal model. Sheep weighing about 60-80 kg each are chosen. Several of the animals will undergo chondrocyte harvesting procedure prior to implantation. The harvested chondrocytes are expanded and seeded onto plasma protein matrices prepared from human plasma. An exemplary study design is shown below in table 4.

The experiments are performed in accordance with the principles of the local laws for Animal Experiments. The animals are examined for evidence of disease or lameness. Acceptability into the study is contingent on being disease free, clinically sound, and no history of prior use. Osteoarthritis is excluded by a preoperative X-ray. The animals are conditioned for an appropriate period of time. A unique number tattoo and ear tag identified each animal. Animals are assigned to the treatment groups by random allocation of identification numbers.

**Table 4: Experimental setup**

| **# Sheep** | **Treatment** |
|---|---|
| 1A-4A | untreated |
| 1B-4B | microfracture |
| 1C-4C | Matrix (20 mg/ml) |
| 5C-8C | Matrix (24 mg/ml) |
| 9C-12C | Matrix (28 mg/ml) |
| 1D-4D | Cell bearing Matrix (20 mg/ml) |
| 5D-8D | Cell bearing Matrix (24 mg/ml) |
| 9D-12D | Cell bearing Matrix (28 mg/ml) |

*Group A. Untreated defects:* 4 animals (8 defects) the chondral defects are left untreated.

*Group B. Microfracture:* 4 animals (8 defects) microfracture is performed without further treatment.

*Group C Plasma protein matrix alone:* Plasma protein matrices, different concentrations, comprising 0.01% HA are implanted in 12 sheep (1C-12C).

*Group D Cell bearing plasma protein matrix:* Chondrocyte bearing matrices comprising cross HA are implanted in 12 sheep (1D-12D).

The attending veterinarian will perform a clinical diagnosis and treatment on the animal if it shows signs of illness. Bodyweight measurements are taken once during the quarantine period, prior to surgery (Day 0) and at the end of the study (Day 112).

***Operation:*** The left knee joint is sterilely draped and opened by an anteromedial approach under general anaesthesia. The medial condyle is exposed, and small pieces of cartilage were harvested from the low weight bearing surfaces of the trochlea and intercondylar notch. The cartilage is cut superficially with a scalpel to avoid bleeding. The wound closure is performed in layers. An external plaster fixation for stifle joint and ankle is applied for five days and cage activity limited to reduce joint loading in order to prevent dislodgement of the patella. The tissue specimen is diced and washed under sterile conditions and the cells isolated by collagenase following a standard digestion protocol. The cells were plated in 75 ml flasks (Coming) and incubated at 37° C. Changing of media is performed every other day. After 2-3 weeks about 200,000 (2x10⁵) cells were seeded on the plasma [protein matrices and cultivated for 3-4 days in 6-well plates. The cell-bearing matrices are sterilely transferred to the operation room. The medial condyle of the right knee of the same sheep is exposed. Using a 4.5-mm punch (Smith & Nephew), two defects, 1 and 2.5 cm distal from the intercondylar notch, are made in the medial condyle of the femur. The defects are outlined with the dermal punch down to the subchondral bone and the cartilage is removed with small curettes. The matrices are fixed into place using fibrin glue.

After treatment of the defect, bleeding points of the capsule are stopped by cauterization and wound closure performed in layers. The external plaster fixation is applied for another five days and cage activity limited to reduce joint loading in order to prevent dislodgment of the graft and reparative tissue. After removal of the plaster, the sheep are given unrestricted activity in runs, and fed with a balanced nutrition twice a day. Until the second postoperative day 2g cefazolin is administered thrice daily.

Necropsy: Animals will be humanely sacrificed at 16 weeks postoperatively according to the guidelines set forth by the AVMA Panel on Euthanasia.

Gross evaluation and sample collection is performed. The articulating surfaces opposing the defect sites are examined for any abnormal joint surface. Additionally, gross evaluations of the knee joints are made to determine the cartilage repair based on previous scoring criteria listed in table 5 below. Femora, patellae, synovium, and popliteal lymph nodes shall be harvested and placed into appropriately labeled containers. Immediately following tissue harvest, gross morphological examination of the cartilage surface was performed and photographic records made of each specimen.

Histology and Histological Evaluation: The knees are opened under sterile conditions and a culture swab obtained. Synovium is documented macroscopically and the defects are photographed and the joint grossly examined. The distal femur is removed and placed in 10% neutral buffered formalin for 12 hours. Areas of trochlea containing the defects and the harvest sites are dissected and placed into 10% formalin for 4 days. The specimens are subsequently placed into a decalcification solution [100g Tritriplex (Epignost, Austria) and 33g Tris-hydroxymethylene-amnomethane (Merck Eurolab, Belgium) per liter] for two to four days at room temperature. The decalcified specimens are embedded in paraffin and cut in a microtome to 5 µm thick sections.

**Table 5: Scoring Criteria for Gross Morphological Evaluations**

| ***Characteristic*** | ***Grading*** | ***Score*** |
|---|---|---|
| Edge Integration (new tissue relative to native cartilage) | Full | 2 |
| | Partial | 1 |
| | None | 0 |
| Smoothness of the cartilage surface | Smooth | 2 |
| | Intermediate | 1 |
| | Rough | 0 |
| Cartilage surface, degree of filling | Flush | 2 |
| | Slight depression | 1 |
| | Depressed/overgrown | 0 |
| Color of cartilage, opacity or translucency of the neocartilage | Transparent | 2 |
| | Translucent | 1 |
| | Opaque | 0 |

Sections are stained with hematoxylin and eosin (H&E), safranin O/ Fast Green, alcian blue and azan for evaluation of tissue types. Immunohistochemistry with antibodies for type I and type II collagens is performed according to a standard ABC protocol using HRP conjugated antibodies. Normal healthy ovine cartilage and tendon served as controls.

Light microscopy is performed on a Vanox Olympus research microscope implementing a histomorphometric method to determine the percentage of selected tissue types (analySiS). Multiple serial transverse histological sections from the middle portion of the defect are evaluated. The filling of the defect is determined as an area percentage of reparative tissue in the defect, based on the cross-sectional area in a sagittal plane through the center of the lesion. The area of the defect, of the filling, height and base of the defect, and tissue type are evaluated. The tissue types are characterized as follows: 1. fibrous tissue 2. transitional tissue 3. hyaline tissue and 4. articular cartilage. Semiquantitative analysis of the defect and adjacent tissue are done according standard scores adapted from O'Driscoll, Pineda and Frenkel.

### Example 12: Human Clinical Trial

A feasibility study to evaluate the safety and performance of the plasma protein matrices of the invention in the treatment of chronic cartilage defects of the femoral condyle will be submitted to the authorities.

A phase I, non-randomized, open label, safety study using a plasma protein matrix or a cell-bearing plasma protein matrix of the present invention and autologous chondrocyte in patients is performed. Patients meeting the entrance criteria will undergo an arthroscopic procedure to confirm diagnosis and to harvest a biopsy for the growth of chondrocytes for future transplantation. Three to six weeks following cell harvest, patients will be hospitalized for surgery. After surgery, patients will be monitored for safety as follows: during 5-7 days hospitalization; after discharge at week 2 and week 6, and performance evaluation at week 12, month 6, and month 12.

The primary endpoint is to evaluate the safety the matrix serves as a scaffold for the seeding and transplantation of autologous chondrocytes in the treatment of a chronic cartilage condyle lesion. The secondary endpoint is to evaluate the performance of a cell-bearing matrix in restoring function, as measured by an improvement in: MRI scores, quality of life questionnaire, joint function score. The safety parameters will include vital signs, serum chemistry, hematology and systemic and local adverse events.

### Example 13: One-Step Procedure for Treating Damaged Cartilage:

Autologous chondrocyte transplantation (ACT) has proven clinically effective in restoring hyaline-like cartilage to isolated chondral defects of the knee. The technique requires three major steps: 1) diagnostic arthroscopy and biopsy of healthy cartilage, 2) cell cultivation, 3) injection of cultured chondrocytes into the lesion under a periosteal flap, which is taken from the tibia and sutured over the lesion.

The disadvantages of ACT include the need for two separate surgical procedures, the requirement for a second site surgery to isolate a periosteal flap and the tendency for cartilage overgrowth due to the presence of the flap. The procedure has gained limited acceptance in the orthopedic community due to the laborious surgical procedure and lengthy rehabilitation. An improved variation provides implantation of a matrix (autologous or allogeneic) of the present invention in a less traumatic method such a hemiarthrotomy or arthroscopy and avoiding the extra surgical step and trauma associated with the periosteal flap. Additionally, an individual may donate plasma several days prior to the surgery for preparation of an autologous matrix.

Kit: A kit comprising the components for practicing the method of the invention, will allow for the convenient practice of the method of the invention in a surgical setting. In one embodiment, a kit will provide sterile components suitable for use in the surgical setting including, sterile solutions (saline, enzymes) a cell-free or cell-bearing matrix suitable for supporting autologous chondrocytes that are to be implanted into an articular joint surface defect and instructions for use.

### Example 14: Bone Repair Model

The plasma protein matrix of the present invention is useful for the treatment of bone defects including osteotomy, particularly in non-weight bearing regions of the skeleton. Suitable animal models include a 4-6 mm osteotomy in the mid ulna bone of rabbits. The ulna is chosen because it is only slightly weight-bearing and allows the creation of a bone defect without requiring a cast or other immobilization treatment. The surgical procedure includes standard anesthesia protocols.. A sponge of the invention is placed into the gap area in each limb and the fracture is closed. Animals are treated with analgesic for 3 days post operation. The duration of the experiment is 6 weeks.

Healing time and quality assessment: X-ray grading provides fracture healing status assessment. Rabbits are X-rayed every other week for 5-6 weeks after surgery. X-rays are scored by two orthopedic surgeons in a blinded manner according to a standard grading scale protocol. At the end of the experiment, rabbits are sacrificed and fracture area is sent for histological and mechanical strength evaluation. Histology is scored by a pathologist using standard staining methods, using hematoxylin and eosin (H&E) for cytoplasm and nucleus and indigo-carmin staining for detection of newly generated callus. Mechanical strength evaluation is performed using the "4 points bending" method.

The treatments groups are: sham osteotomy, osteotomy treated with plasma protein sponge alone, osteotomy treated with plasma protein sponge comprising glycosaminoglycan, osteotomy treated with a plasma protein sponge comprising glycosaminoglycan, optional heparin growth factors.

Another example of an animal model for bone repair is presented in Cook et al, (Am J. Vet Res 64:2-20, 2003).

## Claims

1. A porous, freeze-dried plasma protein matrix comprising plasma proteins and thrombin, having two opposing surfaces substantially parallel to the horizontal axis of the matrix and at least one additional surface extending along the periphery of the sponge substantially parallel to the vertical axis, wherein the plasma proteins crosslinked by the action of thrombin are present in a gradient having a higher concentration along one of the opposing surfaces, and wherein the average size of the pores is smaller along the surface of the matrix exposed to the higher concentration of thrombin.

2. The porous freeze-dried plasma protein matrix according to claim 1,
Wherein the thrombin is provided at a concentration of 300 IU/ml to 1,500 IU/ml; or
wherein the thrombin is provided at a concentration of 500 IU/ml to 1000 IU/ml; or
wherein the plasma proteins and thrombin are present in a ratio of 5:1 (v/v) to 50:1 (v/v); or
wherein the plasma proteins and thrombin are present in a ratio of 8:1 (v/v) to 30:1 (v/v); or
wherein the pores having an average size of 5 µm to 30 µm in cross section in the fraction of the matrix exposed to the higher concentration of thrombin; or
wherein the pores having an average size of 10 µm to 20 µm in cross section in the fraction of the matrix exposed to the higher concentration of thrombin; or
wherein the plasma proteins are substantially devoid of plasminogen; or
wherein the plasma proteins are substantially devoid of organic chelating agents; or
wherein at least one of the plasma proteins is autologous; or
wherein at least one of the plasma proteins is recombinant; or
further comprising a fibrin fiber-modifying agent; or
having any suitable geometric shape; or
wherein the plasma proteins crosslinked by the action of thrombin are generated by a thrombin solution comprising a transglutaminase; preferably wherein the transglutaminase is factor XIII.

3. The porous freeze-dried plasma protein matrix according to claim 1, wherein the plasma proteins crosslinked by the action of thrombin are generated by a thrombin solution comprising at least one viscosity-enhancing agent selected from a protein, a glycolsamino-glycan, a polysaccharide, disaccharide and a synthetic polymer.

4. The porous freeze-dried plasma protein matrix according to claim 3, wherein the protein is an extracellular matrix protein selected from collagen, fibronectin, elastin and laminin.

5. The porous freeze-dried plasma protein matrix according to claim 4,
wherein the protein is collagen; or
wherein the polypeptide is fibronectin.

6. The porous freeze-dried plasma protein matrix according to claim 3, wherein the glycosaminoglycan is selected from crosslinked hyaluronic acid and non- crosslinked hyaluronic acid.

7. The porous freeze-dried plasma protein matrix according to claim 1 further comprising at least one additive selected from the group consisting of a polysaccharide, a glycosaminoglycan and a synthetic polymer.

8. The porous freeze-dried plasma protein matrix according to claim 7,
wherein the glycosaminoglycan is selected from crosslinked hyaluronic acid, non-crosslinked hyaluronic acid and heparin and derivatives thereof.

9. The porous freeze-dried plasma protein matrix according to claim 1 or 7 further comprising at least one bioactive agent selected from the group consisting of growth factors, cytokines, platelets, platelet supernatant and platelet derived proteins, hormones, analgesics, anti-inflammatory agents, anti-microbials and enzymes.

10. The porous freeze-dried plasma protein matrix according to claim 9, wherein the growth factor is selected from a fibroblast growth factor and variant thereof.

11. The porous freeze-dried plasma protein matrix according to claim 1 further comprising cells.

12. The porous freeze-dried plasma protein matrix according to claim 11, wherein the cells are selected from stem cells, progenitor cells, chondrocytes, osteoblasts, hepatocytes, mesenchymal cell types, endothelial cell types, epithelial cell types, urothelial cell types, endocrinal cell types, neuronal cell types, pancreatic cell types, renal cell types and ocular cell types.

13. The porous freeze-dried plasma protein matrix according to claim 12,
wherein the cells are chondrocytes; or
wherein the cells are hepatocytes.

14. A method of preparing a porous, freeze-dried plasma protein matrix comprising plasma proteins and thrombin, having two opposing surfaces substantially parallel to the horizontal axis of the matrix and at least one additional surface extending along the periphery of the sponge substantially parallel to the vertical axis, wherein the plasma proteins crosslinked by the action of thrombin are present in a gradient having a higher concentration along one of the opposing surfaces and, wherein the average size of the pores in cross section is smaller along the surface of the matrix exposed to the higher concentration of thrombin comprising the steps:
introducing a thrombin solution to a solid receptacle or mold;
layering a plasma protein solution over the thrombin solution in the solid receptacle or mold;
incubating under conditions appropriate to achieve clotting;
freezing the clotted mixture; and
lyophilizing the clotted mixture, to obtain a porous matrix.

15. The method according to claim 14,
further comprising the steps of: seeding the porous matrix with cells; and implanting said cell-bearing porous matrix into an individual in need thereof; or
wherein the thrombin solution comprises thrombin at a concentration of 300 IU/ml to 1,500 IU/ml; or
wherein the thrombin the thrombin solution comprises thrombin at a concentration 500 IU/ml to 1000 IU/ml; or
wherein the plasma protein solution and thrombin solution are provided in a ratio of 5:1 (v/v) to 50:1 (v/v); or
wherein the plasma protein solution is substantially devoid of plasminogen; or wherein the plasma protein solution is substantially devoid of organic chelating agents; or
wherein at least one of the plasma proteins is autologous; or
wherein at least one of the plasma proteins is recombinant; or
wherein said porous freeze-dried plasma protein matrix further comprises an agent affecting fibrin fiber thickness, wherein the agent is selected from the group consisting of: calcium ions, a salt that alters ionic strength, a serine protease activator, a serine protease inhibitor and dextran sulfate; or
wherein the receptacle or mold having any suitable shape.

16. The method according to claim 14 wherein the plasma protein solution and thrombin solution are provided in a ratio of 8:1 (v/v) to 30: 1 (v/v).

17. The method according to claim 16, the thrombin solution further comprising at least one viscosity-enhancing agent selected from a protein, a glycosaminoglycan, a polysaccharide, disaccharide and a synthetic polymer.

18. The method according to claim 17, wherein the protein is an extracellular matrix protein selected from collagen fibronectin, elastin and laminin.

19. The method according to claim 18,
wherein the protein is collagen; or
wherein the polypeptide is fibronectin.

20. The method according to claim 17, wherein the glycosaminoglycan is selected from crosslinked hyaluronic acid and non-crosslinked hyaluronic acid.

21. The method according to claim 14, the thrombin solution comprising a transglutaminase, wherein the transglutaminase is preferably factor XIII.

22. The method according to claim 14, the plasma protein solution further comprising at least one additive selected from the group consisting of a polysaccharide, a glycosaminoglycan and a synthetic polymer.

23. The method according to claim 22 wherein the at least one additive is a glycosaminoglycan.

24. The method according to claim 23,
wherein said glycosaminoglycan is selected from crosslinked and non-crosslinked hyaluronic acid; or
wherein said glycosaminoglycan is selected from heparin and a derivative thereof.

25. The method according to claim 14 or claim 22, the plasma protein solution further comprising at least one bioactive agent selected from the group consisting of growth factors, cytokines, platelets, platelet supernatant and platelet derived proteins, hormones, analgesics, anti-inflammatory agents, anti-microbials and enzymes.

26. The method according to claim 25, wherein the growth factor is selected from a fibroblast growth factor and variant thereof.

27. The method according to claim 15, wherein when the method further comprises the steps of: seeding the porous matrix with cells; and implanting said cell-bearing porous matrix into an individual in need thereof, the cells are selected from stem cells, progenitor cells, chondrocytes, osteoblasts, hepatocytes, mesenchymal cell types, endothelial cell types, epithelial cell types, urothelial cell types, endocrinal cell types, neuronal cell types, pancreatic cell types, renal cell types and ocular cell types.

28. The method according to claim 27,
wherein the cells are chondrocytes; or
wherein the cells are hepatocytes.

29. A porous, freeze-dried plasma protein matrix according to any of the claims 1 13 for use in the treatment of diseased or injured tissue.

30. The protein matrix according to claim 29,
wherein the diseased or injured tissue is selected from neural, muscular, skeletal, cartilaginous, tendonous, hepatic, pancreatic, renal, ocular, arteriovenous, mammary and urinary tissue types; preferably cartilage tissue or hepatic tissue.

## Patentansprüche

1. Poröse, gefriergetrocknete Plasmaprotein-Matrix, welche Plasmaproteine und Thrombin umfasst, und zwei gegenüberliegende Oberflächen aufweist, die im Wesentlichen parallel zu der Horizontalachse der Matrix verlaufen, und mindestens eine weitere Oberfläche, die sich um den Umfang des Schwamms im Wesentlichen parallel zu der vertikalen Achse erstreckt, worin die mittels Thrombin vernetzten Plasmaproteine in einem Gradienten vorhanden sind, der entlang einer der gegenüberliegenden Oberflächen eine höhere Konzentration aufweist, und worin die durchschnittliche Porengrösse entlang der Oberfläche der Matrix, die der höheren Thrombinkonzentration ausgesetzt ist, kleiner ist.

2. Poröse, gefriergetrocknete Plasmaprotein-Matrix nach Anspruch 1,
worin das Thrombin in einer Konzentration von 300 IU/ml bis 1500 IU/ml bereit gestellt ist, oder
worin das Thrombin in einer Konzentration von 500 IU/ml bis 1000 IU/ml bereit gestellt ist, oder
worin die Plasmaproteine und Thrombin in einem Verhältnis von 5:1 (Vol./Vol.) bis 50:1 (Vol./Vol.) vorhanden sind, oder
worin die Plasmaproteine und Thrombin in einem Verhältnis von 8:1 (Vol./Vol.) bis 30:1 (Vol./Vol.) vorhanden sind, oder
worin die Poren in der Fraktion der Matrix, die der höheren Konzentration Thrombin ausgesetzt waren, eine durchschnittliche Querschnittsgröße von 5 µm bis 30 µm aufweisen, oder
worin die Plasmaproteine im Wesentlichen frei von Plasminogen sind, oder
worin die Plasmaproteine im Wesentlichen frei von organischen Chelatmitteln sind, oder
worin mindestens eines der Plasmaproteine autolog ist, oder
worin mindestens eines der Plasmaproteine rekombinant ist, oder
weiter umfassend ein Fibrinfaser-modifizierendes Mittel, oder
jede geeignete geometrische Form aufweisend, oder
worin die mittels Thrombin vernetzten Plasmaproteine aus einer Thrombinlösung erzeugt werden, welche eine Transglutaminase umfasst, vorzugsweise worin die Transglutaminase Faktor XIII ist.

3. Poröse, gefriergetrocknete Plasmaprotein-Matrix nach Anspruch 1, worin die mittels Thrombin vernetzten Plasmaproteine aus einer Thrombinlösung erzeugt werden, welche mindestens ein Viskositäts-steigerndes Mittel umfasst, ausgewählt unter einem Protein, einem Glycosaminoglycan, einem Polysaccharid, Disaccharid und einem synthetischen Polymer.

4. Poröse, gefriergetrocknete Plasmaprotein-Matrix nach Anspruch 3, worin das Protein ein extrazelluläres Matrixprotein ist, ausgewählt unter Kollagen, Fibronectin, Elastin und Laminin.

5. Poröse, gefriergetrocknete Plasmaprotein-Matrix nach Anspruch 4,
worin das Protein Kollagen ist, oder
worin das Polypeptid Fibronectin ist.

6. Poröse, gefriergetrocknete Plasmaprotein-Matrix nach Anspruch 3, worin das Glycosaminoglycan ausgewählt ist unter vernetzter Hyaluronsäure und nicht-vernetzter Hyaluronsäure.

7. Poröse, gefriergetrocknete Plasmaprotein-Matrix nach Anspruch 1, welche weiter mindestens einen Zusatz umfasst, ausgewählt aus der Gruppe bestehend aus einem Polysaccharid, einem Glycosaminoglycan und einem synthetischen Polymer.

8. Poröse, gefriergetrocknete Plasmaprotein-Matrix nach Anspruch 7,
worin das Glycosaminoglycan ausgewählt ist unter Hyaluronsäre, vernetzter Hyaluronsäure, nicht-vernetzter Hyaluronsäre und Heparin und Derivaten davon.

9. Poröse, gefriergetrocknete Plasmaprotein-Matrix nach Anspruch 1 oder 7, welche weiter mindestens ein bioaktives Mittel umfasst, ausgewählt aus der Gruppe bestehend aus Wachstumsfaktoren, Zytokinen, Platelets, Platelet-Überstand und Platelet-abgeleiteten Proteinen, Hormonen, Analgetika, anti-entzündlichen Mitteln, anti-mikrobiellen Mitteln und Enzymen.

10. Poröse, gefriergetrocknete Plasmaprotein-Matrix nach Anspruch 9, worin der Wachstumsfaktor ausgewählt ist unter einem Fibroblasten-Wachstumsfaktor und Varianten davon.

11. Poröse, gefriergetrocknete Plasmaprotein-Matrix nach Anspruch 1, welche weiter Zellen umfasst.

12. Poröse, gefriergetrocknete Plasmaprotein-Matrix nach Anspruch 11, worin die Zellen ausgewählt sind unter Stammzellen, Vorläuferzellen, Chondrozyten, Osteoblasten, Hepatozyten, Mesenchymal-Zelltypen, Endothel-Zelltypen, Epithel-Zelltypen, Urothel-Zellytpen, endokrinen Zelltypen, neuronalen Zelltypen, Pankreas-Zelltypen, renalen Zelltypen und okularen Zelltypen.

13. Poröse, gefriergetrocknete Plasmaprotein-Matrix nach Anspruch 12,
worin die Zellen Chondrozyten sind, oder
worin die Zellen Hepatozyten sind.

14. Verfahren zur Herstellung einer porösen, gefriergetrockneten Plasmaprotein-Matrix, welche Plasmaproteine und Thrombin umfasst, zwei gegenüberliegende Oberflächen aufweist, die im Wesentlichen parallel zu der Horizontalachse der Matrix verlaufen und mindestens eine weitere Oberfläche, die sich um den Umfang des Schwamms im Wesentlichen parallel zu der vertikalen Achse erstreckt, worin die mittels Thrombin vernetzten Plasmaproteine in einem Gradienten vorhanden sind, der entlang einer der gegenüberliegende Oberflächen eine höhere Konzentration aufweist, und worin die durchschnittliche Querschnitts-Porengrösse entlang der Oberfläche der Matrix, die der höheren Thrombinkonzentration ausgesetzt ist, kleiner ist, welches die Schritte umfasst:
Einbringen einer Thrombinlösung in einen festen Behälter oder Gussform,
Schichten einer Plasmaproteinlösung über die Thrombinlösung in dem festen Behälter oder Gussform,
Inkubieren unter Bedingungen, die geeignet sind Koagulation hervorzurufen,
Einfrieren des koagulierten Gemisches,
Lyophilisieren des koagulierten Gemisches, um eine poröse Matrix zu erhalten.

15. Verfahren nach Anspruch 14,
weiter umfassend die Schritte: Aussähen von Zellen in der porösen Matrix, und Implantieren der Zell-tragenden Matrix in ein Individuum, das diese benötigt, oder
wobei die Thrombinlösung Thrombin in einer Konzentration von 300 IU/ml bis 1500 IU/ml umfasst, oder
wobei die Thrombinlösung Thrombin in einer Konzentration von 500 IU/ml bis 1000 IU/ml umfasst, oder
worin die Plasmaproteinlösung und Thrombinlösung in einem Verhältnis von 5:1 (Vol./Vol.) bis 50:1 (Vol./Vol.) bereit gestellt werden, oder
wobei die Plasmaproteinlösung im Wesentlichen frei von Plasminogen ist, oder wobei die Plasmaproteinlösung im Wesentlichen frei von organischen Chelatmitteln ist, oder
wobei mindestens eines der Plasmaproteine autolog ist, oder
wobei mindestens eines der Plasmaproteine rekombinant ist, oder
wobei die poröse gefriergetrocknete Plasmaprotein-Matrix weiter ein Mittel umfasst, das die Fibrinfaser-Dicke beeinflusst, wobei das Mittel ausgewählt ist aus der Gruppe bestehend aus: Calciumionen, einem Salz, das die Ionenstärke verändert, einen Serinprotease-Aktivator, einem Serinprotease-Inhibitor und Dextransulfat, oder
wobei der Behälter oder die Gussform jede geeignete Form aufweist.

16. Verfahren nach Anspruch 14, wobei die Plasmaproteinlösung und Thrombinlösung in einem Verhältnis von 8:1 (Vol./Vol.) bis 30:1 (Vol./Vol.) bereit gestellt werden.

17. Verfahren nach Anspruch 16, wobei die Thrombinlösung weiter mindestens ein Viskositäts-steigerndes Mittel umfasst, ausgewählt unter einem Protein, einem Glycosaminoglycan, einem Polysaccharid, Disaccharid und einem synthetischen Polymer.

18. Verfahren nach Anspruch 17, wobei das Protein ein extrazelluläres Matrixprotein ist ausgewählt unter Kollagen, Fibronectin, Elastin und Laminin.

19. Verfahren nach Anspruch 18,
wobei das Protein Kollagen ist, oder
wobei das Polypeptid Fibronectin ist.

20. Verfahren nach Anspruch 17, wobei das Glycosaminoglycan ausgewählt ist unter vernetzter Hyaluronsäure und nicht-vernetzter Hyaluronsäure.

21. Verfahren nach Anspruch 14, wobei die Thrombinlösung eine Transglutaminase umfasst, wobei die Transglutaminase vorzugsweise Faktor XIII ist.

22. Verfahren nach Anspruch14, wobei die Plasmaproteinlösung weiter mindestens einen Zusatz umfasst, ausgewählt aus der Gruppe bestehend aus einem Polysaccharid, einem Glycosaminoglycan und einem synthetischen Polymer.

23. Verfahren nach Anspruch 22, wobei der mindestens eine Zusatz ein Glycosaminoglycan ist.

24. Verfahren nach Anspruch 23,
wobei das Glycosaminoglycan ausgewählt ist unter vernetzter und nicht-vernetzter Hyaluronsäure, oder
wobei das Glycosaminoglycan ausgewählt ist unter Heparin und einem Derivat davon.

25. Verfahren nach Anspruch 14 oder Anspruch 22, wobei die Plasmaproteinlösung weiter mindestens ein bioaktives Mittel umfasst, ausgewählt aus der Gruppe bestehend aus Wachstumsfaktoren, Zytokinen, Platelets, Platelet-Überstand und Platelet-abgeleiteten Proteinen, Hormonen, Analgetika, anti-entzündlichen Mitteln, anti-mikrobiellen Mitteln und Enzymen.

26. Verfahren nach Anspruch 25, wobei der Wachstumsfaktor ausgewählt ist unter Fibroblasten-Wachstumsfaktor und Varianten davon.

27. Verfahren nach Anspruch 15, wobei dann, wenn das Verfahren die Schritte umfasst: Aussähen von Zellen in der porösen Matrix, und Implantieren der Zell-tragenden Matrix in ein Individuum, das diese benötigt, die Zellen ausgewählt sind unter Stammzellen, Vorläuferzellen, Chondrozyten, Osteoblasten, Hepatozyten, Mesenchymal-Zelltypen, Endothel-Zelltypen, Epithel-Zelltypen, Urothel-Zellytpen, endokrinen Zelltypen, neuronalen Zelltypen, Pankreas-Zelltypen, renalen Zelltypen und okularen Zelltypen.

28. Verfahren nach Anspruch 27,
wobei die Zellen Chondrozyten sind, oder
wobei die Zellen Hepatozyten sind.

29. Poröse, gefriergetrocknete Plasmaprotein-Matrix nach einem der Ansprüche 1 - 13 zur Verwendung bei der Behandlung von erkranktem oder abgestorbenem Gewebe.

30. Poröse, gefriergetrocknete Plasmaprotein-Matrix nach Anspruch 29,
worin das erkrankte oder abgestorbene Gewebe ausgewählt ist unter neuralem, Muskel-, Skelett-, Knorpel-, Sehnen-, Leber-, Pankreas-, renalem, okularem, arteriovenösem, Brust- und Blasen-Gewebetypen, vorzugsweise Knorpel-Gewebe oder Leber-Gewebe.

## Revendications

1. Une matrice poreuse lyophilisée de protéines plasmatiques comprenant des protéines plasmatiques et de la thrombine, comportant deux surfaces opposées sensiblement parallèles à l'axe horizontal de la matrice et au moins une surface additionnelle s'étendant le long de la périphérie de l'éponge de manière sensiblement parallèle à l'axe vertical, dans laquelle les protéines plasmatiques réticulées par l'action de la thrombine sont présentes selon un gradient ayant une concentration plus élevée le long d'une des surfaces opposées et dans laquelle la taille moyenne des pores est plus petite le long de la surface de la matrice exposée à la concentration de thrombine la plus élevée.

2. La matrice poreuse lyophilisée de protéines plasmatiques selon la revendication 1, dans lequel la thrombine est fournie à une concentration de 300 UI / ml à 1 500 UI / ml ; ou
dans laquelle la thrombine est fournie à une concentration de 500 UI / ml à 1 000 UI / ml ; ou
dans laquelle les protéines plasmatiques et la thrombine sont présentes dans un rapport de 5:1 (v/v) à 50:1 (v/v) ; ou
dans laquelle les protéines plasmatiques et la thrombine sont présentes dans un rapport de 08:1 (v/v) à 30:1 (v/v) ; ou
dans laquelle les pores ont une taille moyenne de 5 µm m à 30 µm m en section transversale
dans la portion de matrice exposée à la plus forte concentration de thrombine ; ou
dans laquelle les pores ont une taille moyenne de 10 µm m à 20 µm en section transversale
dans la portion de matrice exposée à la plus forte concentration de thrombine ; ou
dans laquelle les protéines plasmatiques sont sensiblement dépourvues de plasminogène ; ou
dans laquelle les protéines plasmatiques sont sensiblement dépourvues d'agents de chélation organiques ; ou
dans laquelle au moins une des protéines plasmatiques est autologue ; ou
dans laquelle au moins une des protéines plasmatiques est recombinante ; ou
comprenant en outre un agent modificateur des fibres de fibrine ; ou
ayant une forme géométrique convenable ; ou
dans laquelle les protéines plasmatiques réticulées par l'action de la thrombine sont produites à l'aide d'une solution de thrombine comprenant une transglutaminase, de préférence dans laquelle la transglutaminase est le facteur XIII.

3. La matrice poreuse lyophilisée de protéines plasmatiques selon la revendication 1, dans laquelle les protéines plasmatiques réticulées par l'action de la thrombine sont produites à l'aide d'une solution de thrombine comprenant au moins un agent améliorant la viscosité agent choisi parmi une protéine, un glycosamino-glycane, un polysaccharide, un disaccharide et un polymère synthétique.

4. La matrice poreuse lyophilisée de protéines plasmatiques selon la revendication 3, dans laquelle la protéine est une protéine de matrice extracellulaire choisie parmi le collagène, la fibronectine, l'élastine et la laminine.

5. La matrice poreuse lyophilisée de protéines plasmatiques selon la revendication 4, dans laquelle la protéine est le collagène ; ou
dans laquelle le polypeptide est la fibronectine.

6. La matrice de protéine plasmatique lyophilisée poreux selon la revendication 3, dans laquelle le glycosamino-glycane est choisi parmi l'acide hyaluronique réticulé et l'acide hyaluronique non réticulé.

7. La matrice poreuse lyophilisée de protéines plasmatiques selon la revendication 1, comprenant en outre au moins un additif choisi dans le groupe consistant en un polysaccharide, un glycosamino-glycane et un polymère synthétique.

8. La matrice poreuse lyophilisée de protéines plasmatiques selon la revendication 7, dans laquelle le glycosamino-glycane est choisi parmi l'acide hyaluronique réticulé, l'acide hyaluronique non réticulé et l'héparine et ses dérivés.

9. La matrice poreuse lyophilisée de protéines plasmatiques selon la revendication 1 ou 7, comprenant en outre au moins un agent bioactif choisi parmi le groupe constitué par les facteurs de croissance, cytokines, plaquettes, protéines de surnageant de plaquettes et de dérivé de plaquettes, hormones, analgésiques, agents anti-inflammatoires, antimicrobiens et enzymes.

10. La matrice poreuse lyophilisée de protéines plasmatiques selon la revendication 9, dans laquelle le facteur de croissance est choisi parmi un facteur de croissance des fibroblastes et la variante de celui-ci.

11. La matrice poreuse lyophilisée de protéines plasmatiques selon la revendication 1, comprenant en outre des cellules.

12. La matrice poreuse lyophilisée de protéines plasmatiques selon la revendication 11, dans laquelle les cellules sont sélectionnées à partir de cellules souches, cellules germinatives, chondrocytes, ostéoblastes, hépatocytes, des types de cellules mésenchymateuses, des types de cellules endothéliales, des types de cellules épithéliales, des types de cellules urothéliales, des types de cellules endocrines, des types de cellules neuronales, des types de cellules pancréatiques, des types de cellules rénales et de types de cellules oculaires.

13. La matrice poreuse lyophilisée de protéines plasmatiques selon la revendication 12, dans laquelle les cellules sont des chondrocytes ; ou
dans laquelle les cellules sont des hépatocytes.

14. Procédé de préparation d'une matrice poreuse lyophilisée de protéines plasmatiques comprenant des protéines plasmatiques et de la thrombine, comportant deux surfaces opposées sensiblement parallèles à l'axe horizontal de la matrice et au moins une surface additionnelle s'étendant le long de la périphérie de l'éponge sensiblement parallèlement à l'axe vertical, dans lequel les protéines plasmatiques réticulées par l'action de la thrombine sont présentes selon un gradient ayant une concentration plus élevée le long d'une des surfaces opposées et dans lequel la taille moyenne des pores est plus petite en section transversale le long de la surface de la matrice exposée à la concentration de thrombine la plus élevée, comprenant les étapes consistant à:
introduire une solution de thrombine dans un récipient solide ou un moule;
déposer une solution de protéines plasmatiques sur la solution de thrombine dans le récipient solide ou le moule;
incuber dans des conditions appropriées à réaliser la coagulation;
congeler le mélange coagulé, et
lyophiliser le mélange coagulé pour obtenir une matrice poreuse.

15. Le procédé selon la revendication 14, comprenant en outre les étapes de:
ensemencer la matrice poreuse avec des cellules et implanter ladite matrice poreuse contenant des cellules dans un individu en ayant besoin ; ou
dans lequel la solution de thrombine comprend de la thrombine dans une concentration de 300 UI / ml à 1'500 UI / ml ; ou
dans lequel la solution de thrombine comprend la thrombine dans une concentration de 500 UI / ml à 1'000 UI / ml ; ou
dans lequel la solution de protéines plasmatiques et la solution de thrombine sont présentes dans un rapport de 5:1 (v/v) à 50:1 (v/v) ; ou
dans lequel la solution de protéines plasmatiques est sensiblement dépourvue de plasminogène ; ou
dans lequel la solution de protéines plasmatiques est sensiblement dépourvue d'agents de chélation organiques ; ou
dans lequel au moins une des protéines plasmatiques est autologue ; ou
dans lequel au moins une des protéines plasmatiques est recombinante ; ou
dans lequel la matrice lyophilisée poreuse de protéines plasmatiques comprend en outre un agent modifiant l'épaisseur des fibres de fibrine, dans lequel l'agent est choisi à partir du groupe consistant en ions calcium, un sel qui influence la force ionique, un activateur de protéase de sérine, un inhibiteur de protéase de sérine et le sulfate de dextrane ; ou
dans lequel le réceptacle ou le moule ont une forme convenable.

16. Le procédé selon la revendication 14 dans lequel la solution de protéines plasmatiques et la solution de thrombine sont présentes dans un rapport de 8:1 (v / v) à 30: 1 (v / v).

17. Le procédé selon la revendication 16 dans lequel la solution de thrombine comprend en outre au moins un agent améliorant la viscosité choisi parmi une protéine, un glycosamino-glycane, un polysaccharide, un disaccharide et un polymère synthétique.

18. Le procédé selon la revendication 17 dans lequel la protéine est une protéine de matrice extracellulaire choisie parmi le collagène, la fibronectine, l'élastine et la laminine

19. Le procédé selon la revendication 18 dans lequel la protéine est le collagène ; ou
dans lequel le polypeptide est la fibronectine.

20. Le procédé selon la revendication 17 dans lequel le glycosamino-glycane est choisi parmi l'acide hyaluronique réticulé et l'acide hyaluronique non réticulé.

21. Le procédé selon la revendication 14 la solution de thrombine comprend une transglutaminase, de préférence dans lequel la transglutaminase est le facteur XIII.

22. Le procédé selon la revendication 14 dans lequel la solution de protéines plasmatiques comprend en outre au moins un additif choisi dans le groupe consistant en un polysaccharide, un glycosamino-glycane et un polymère synthétique.

23. Le procédé selon la revendication 22 dans lequel le au moins un additif est un glycosamino-glycane.

24. Le procédé selon la revendication 23, dans lequel ledit glycosamino-glycane est choisi parmi l'acide hyaluronique réticulé et non réticulé ; ou
dans lequel ledit glycosamino-glycane est choisi parmi l'héparine et un dérivé de celle-ci.

25. Le procédé selon la revendication 14 ou la revendication 22 dans lequel la solution de protéines plasmatiques comprend en outre au moins un agent bioactif choisi dans le groupe constitué par les facteurs de croissance, cytokines, plaquettes, protéines de surnageant de plaquettes et protéines dérivées de plaquettes, hormones, analgésiques, agents anti-inflammatoires, antimicrobiens et enzymes.

26. Le procédé selon la revendication 25, dans lequel le facteur de croissance est choisi parmi un facteur de croissance des fibroblastes et des variantes de celui-ci.

27. Le procédé selon la revendication 15 dans lequel les cellules sont sélectionnées à partir de cellules souches, cellules germinatives, chondrocytes, ostéoblastes, hépatocytes, des types de cellules mésenchymateuses, des types de cellules endothéliales, des types de cellules épithéliales, des types de cellules urothéliales, des types de cellules endocrines, des types de cellules neuronales, des types cellules du pancréas, des types de cellules rénales et types de cellules oculaires.

28. Le procédé selon la revendication 27 dans lequel les cellules sont des chondrocytes ; ou dans lequel les cellules sont des hépatocytes.

29. Une matrice poreuse lyophilisée de protéines plasmatiques selon l'une des revendications 1 - 13 pour utilisation
dans le traitement de tissus malades ou abîmés.

30. La matrice poreuse lyophilisée selon la revendication 29 dans laquelle le tissu malade ou abîmé est choisi parmi les types de tissus neural, musculaire, du squelette, cartilagineux, tendineux, hépatique, pancréatique, rénal, oculaire, artério-veineux, mammaire, de préférence du tissus cartilagineux ou du tissus hépatique.
